(19) Europäisches Patentamt / European Patent Office / Office européen des brevets

(11) **EP 3 941 487 B1**

(12) **EUROPEAN PATENT SPECIFICATION**

(45) Date of publication and mention of the grant of the patent:
**14.02.2024 Bulletin 2024/07**

(21) Application number: **20718095.1**

(22) Date of filing: **19.03.2020**

(51) International Patent Classification (IPC):
**A61K 35/17** *(2015.01)*    **C12N 5/10** *(2006.01)*
**C12N 5/0783** *(2010.01)*    **A61P 35/00** *(2006.01)*

(52) Cooperative Patent Classification (CPC):
**C12N 5/0638; A61K 35/17; A61P 35/00;**
**C12N 5/0636;** C12N 2501/2307; C12N 2501/2315;
C12N 2510/00; C12N 2740/15011

(86) International application number:
**PCT/US2020/023585**

(87) International publication number:
**WO 2020/191172 (24.09.2020 Gazette 2020/39)**

(54) **CD28 T CELL CULTURES, COMPOSITIONS, AND METHODS OF USING THEREOF**

CD28-T-ZELLKULTUREN, ZUSAMMENSETZUNGEN UND VERFAHREN ZU IHRER VERWENDUNG

CULTURES DE CELLULES T CD28, LEURS COMPOSITIONS ET LEURS MÉTHODES D'UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.03.2019 US 201962820442 P**
**28.03.2019 DE 102019108125**

(43) Date of publication of application:
**26.01.2022 Bulletin 2022/04**

(73) Proprietor: **Immatics US, Inc.**
**Houston, TX 77030 (US)**

(72) Inventors:
• **ALPERT, Amir**
**Houston, TX 77030 (US)**
• **KALRA, Mamta**
**Houston, TX 77030 (US)**

(74) Representative: **Zwicker, Jörk**
**ZSP Patentanwälte PartG mbB**
**Hansastraße 32**
**80686 München (DE)**

(56) References cited:
**WO-A1-2015/164745    WO-A1-2017/099712**
**US-B2- 6 905 680**

• **D. TESCHNER ET AL: "In Vitro Stimulation and Expansion of Human Tumour-Reactive CD8+ Cytotoxic T Lymphocytes by Anti-CD3/CD28/CD137 Magnetic Beads : CTL Expansion by CD3/CD28/CD137 Beads In Vitro", SCANDINAVIAN JOURNAL OF IMMUNOLOGY, vol. 74, no. 2, 1 August 2011 (2011-08-01), pages 155-164, XP055452404, GB ISSN: 0300-9475, DOI: 10.1111/j.1365-3083.2011.02564.x**
• **WENG N P ET AL: "CD28^- T cells: their role in the age-associated decline of immune function", TRENDS IN IMMUNOLOGY, ELSEVIER LTD. * TRENDS JOURNALS, GB, vol. 30, no. 7, 1 July 2009 (2009-07-01), pages 306-312, XP026251276, ISSN: 1471-4906, DOI: 10.1016/J.IT.2009.03.013 [retrieved on 2009-06-18]**
• **DIMITRI KASAKOVSKI ET AL: "T cell senescence and CAR-T cell exhaustion in hematological malignancies", JOURNAL OF HEMATOLOGY & ONCOLOGY, vol. 11, no. 1, 4 July 2018 (2018-07-04), XP055701316, DOI: 10.1186/s13045-018-0629-x**

Remarks:
The complete document including Reference Table(s) and the Sequence Listing(s) can be downloaded from the EPO website

**Description**

**BACKGROUND**

1. Field

The present disclosure provides for methods of improving the efficacy of T cells. The disclosure also provides for a genetically transduced T cell population for use in a method of treating cancer.

2. Background

Immunotherapy has emerged as a highly promising approach for treating cancer. Immunotherapy can be subdivided into cellular therapies and small molecule/antibodies therapies. Within the cellular therapy space, chimeric antigen receptor T (CAR-T) cell therapies have shown strong clinical efficacy in liquid tumors, while T-cell receptor T (TCR-T) cell-based therapies have shown promising early results in various solid tumor indications. The efficacy of the clinical products may be driven by their in vivo characteristics, which may be largely imprinted during the ex vivo manufacturing process.

US 8,383,099 describes a method of promoting regression of a cancer in a subject by, for example, by culturing autologous T cells; expanding the cultured T cells using OKT3 antibody, IL-2, and feeder lymphocytes.

US 9,074,185 describes a method of generating a T cell infusion product for promoting regression of a cancer in a subject, including culturing autologous T cells; enriching the cultured T cells for CD8+ T cells; expanding the number of cultured T cells using OKT3 antibody, IL-2, and feeder lymphocytes to provide an expanded number of T cells.

There remains a need to improve the efficacy of T cells and the outcome of ACT in cancer patients. A solution to this technical problem is provided by the embodiments characterized in the claims.

**BRIEF SUMMARY**

In a first aspect, the invention provides a method for producing T cells with improved efficacy for adoptive immunotherapy comprising

obtaining a population of CD8+ T cells from a patient or a donor,
determining a % of CD28+ CD8+ T cells in the obtained population, and
activating the determined population with anti-CD3 antibody and anti-CD28 antibody, provided that the determined population comprises at least about 50% of CD28+ CD8+ T cells, preferably at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% of CD28+ CD8+ T cells, or
activating the determined population with anti-CD3 antibody in the absence of anti-CD28 antibody, provided that the determined population comprises less than about 50% of CD28+ CD8+ T cells, preferably less than about 40%, less than about 35%, less than about 30%, less than about 25%, less than about 20%, less than about 15%, less than about 10%, less than about 9%, less than about 8%, less than about 7%, less than about 6%, less than about 5%, less than about 4%, less than about 3%, less than about 2%, or less than about 1% of CD28+ CD8+ T cells, transducing the activated T cell population with a viral vector, preferably a retroviral or lentiviral vector expressing a T cell receptor (TCR) or a chimeric antigen receptor (CAR), and expanding the transduced T cell population.

In a second aspect, the invention provides a method for producing T cells with improved efficacy for adoptive immunotherapy comprising

obtaining a population of CD8+ T cells from a patient or a donor, and
isolating CD28+ CD8+ T cells from the obtained population,
wherein the isolated cells comprise at least 50% of CD28+ CD8+ T cells, preferably
wherein the isolated cells comprise at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% of CD28+ CD8+ T cells,
activating the isolated cells with anti-CD3 antibody and anti-CD28 antibody,
transducing the activated T cell population with a viral vector, preferably a retroviral or lentiviral vector expressing a T cell receptor (TCR) or a chimeric antigen receptor (CAR), and expanding the transduced T cell population.

In a third aspect, the invention provides a genetically transduced T cell population for use in a method of treating cancer, wherein the method of treating cancer comprises the steps of

determining a % of CD28+ CD8+ T cells in a population of CD8+ T cells obtained from a patient,
activating the determined population with anti-CD3 antibody and anti-CD28 antibody, provided that the determined population comprises at least about 50% of CD28+ CD8+ T cells, or
activating the determined population with anti-CD3 antibody in the absence of anti-CD28 antibody, provided that the determined population comprises less than about 50% of CD28+ CD8+ T cells,
transducing the activated T cell population with a viral vector,
expanding the transduced T cell population, and
administering to the patient the expanded T cell population,
and wherein the cancer is selected from the group consisting of hepatocellular carcinoma (HCC), colorectal carcinoma (CRC), glioblastoma (GB), gastric cancer (GC), esophageal cancer, non-small cell lung cancer (NSCLC), pancreatic cancer (PC), renal cell carcinoma (RCC), benign prostate hyperplasia (BPH), prostate cancer (PCA), ovarian cancer (OC), melanoma, breast cancer, chronic lymphocytic leukemia (CLL), Merkel cell carcinoma (MCC), small cell lung cancer (SCLC), Non-Hodgkin lymphoma (NHL), acute myeloid leukemia (AML), gallbladder cancer and cholangiocarcinoma (GBC, CCC), urinary bladder cancer (UBC), acute lymphoblastic leukemia (ALL), multiple myeloma (MM), and uterine cancer (UEC).

The methods may be ex *vivo* methods for producing T cells with improved efficacy for immunotherapy.

In some embodiments, the transducing and the expanding may be carried out in the presence of at least one cytokine.

In some embodiments, the activating may include immobilizing the T cells with the anti-CD3 antibody and the anti-CD28 antibody on a solid phase support.

In some embodiments, the anti-CD3 antibody and/or the anti-CD28 antibody each have a concentration of from about 0.1 $\mu$g/ml to about 10.0 $\mu$g/ml, about 0.1 $\mu$g/ml to about 8.0 $\mu$g/ml, about 0.1 $\mu$g/ml to about 6.0 $\mu$g/ml, about 0.1 $\mu$g/ml to about 4.0 $\mu$g/ml, about 0.1 $\mu$g/ml to about 2.0 $\mu$g/ml, about 0.1 $\mu$g/ml to about 1.0 $\mu$g/ml, about 0.1 $\mu$g/ml to about 0.5 $\mu$g/ml, about 0.5 $\mu$g/ml to about 10.0 $\mu$g/ml, about 2 $\mu$g/ml to about 8 $\mu$g/ml, about 3 $\mu$g/ml to about 7 $\mu$g/ml, about 2 $\mu$g/ml to about 5 $\mu$g/ml, about 0.5 $\mu$g/ml to about 2.0 $\mu$g/ml, or about 0.5 $\mu$g/ml to about 2.5 $\mu$g/ml.

In some embodiments, the activation may be carried out within a period of from about 1 hour to about 120 hours, about 1 hour to about 108 hours, about 1 hour to about 96 hours, about 1 hour to about 84 hours, about 1 hour to about 72 hours, about 1 hour to about 60 hours, about 1 hour to about 48 hours, about 1 hour to about 36 hours, about 1 hour to about 24 hours, about 2 hours to about 24 hours, about 4 hours to about 24 hours, about 6 hours to about 24 hours, about 8 hours to about 24 hours, about 10 hours to about 24 hours, about 12 hours to about 24 hours, about 12 hours to about 72 hours, about 24 hours to about 72 hours, about 6 hours to about 48 hours, about 24 hours to about 48 hours, about 6 hours to about 72 hours, or about 1 hours to about 12 hours.

In some embodiments, the at least one cytokine may be selected from interleukin (IL)-2, IL-7, IL-10, IL-12, IL-15, IL-21, or combinations thereof.

In some embodiments, the at least one cytokine includes IL-7, IL-15, or a combination of IL-7 and IL-15.

In some embodiments, the concentration of IL-7 is from about 1 ng/ml to 90 ng/ml, about 1 ng/ml to 80 ng/ml, about 1 ng/ml to 70 ng/ml, about 1 ng/ml to 60 ng/ml, about 1 ng/ml to 50 ng/ml, about 1 ng/ml to 40 ng/ml, about 1 ng/ml to 30 ng/ml, about 1 ng/ml to 20 ng/ml, about 1 ng/ml to 15 ng/ml, about 1 ng/ml to 10 ng/ml, about 2 ng/ml to 10 ng/ml, about 4 ng/ml to 10 ng/ml, about 6 ng/ml to 10 ng/ml, or about 5 ng/ml to 10 ng/ml.

In some embodiments, the concentration of IL-15 may be from about 5 ng/ml to 500 ng/ml, about 10 ng/ml to 400 ng/ml, about 15 ng/ml to 300 ng/ml, about 5 ng/ml to 200 ng/ml, about 5 ng/ml to 150 ng/ml, about 5 ng/ml to 100 ng/ml, about 10 ng/ml to 100 ng/ml, about 20 ng/ml to 100 ng/ml, about 30 ng/ml to 100 ng/ml, about 40 ng/ml to 100 ng/ml, about 50 ng/ml to 100 ng/ml, about 60 ng/ml to 100 ng/ml, about 70 ng/ml to 100 ng/ml, about 80 ng/ml to 100 ng/ml, about 90 ng/ml to 100 ng/ml, about 10 ng/ml to 50 ng/ml, about 1 ng/ml to 50 ng/ml, about 5 ng/ml to 50 ng/ml, or about 20 ng/ml to 50 ng/ml.

In some embodiments, the transducing may be carried out within a period of from about 1 hour to 120 hours, about 12 hour to 96 hours, about 24 hour to 96 hours, about 24 hour to 72 hours, about 10 hour to 48 hours, about 1 hour to 36 hours, about 1 hour to 24 hours, about 2 hour to 24 hours, about 4 hour to 24 hours, about 6 hour to 24 hours, about 8 hour to 24 hours, about 10 hour to 24 hours, about 1 hour to 12 hours, about 14 hour to 24 hours, about 1 hour to 12 hours, about 6 to about 18 hours

In some embodiments, the viral vector may be a retroviral vector expressing a T cell receptor (TCR).

In some embodiments, the viral vector may be a lentiviral vector expressing a TCR.

In some embodiments, the expanding may be carried out within a period of from about 1 day to about 30 days, about 5 to about 30 days, about 1 day to about 25 days, about 2 day to about 20 days, about 5 day to about 15 days, about 2 day to about 10 days, about 3 days to about 15 days, about 3 days to about 20 days, about 4 days to about 10 days, about 5 days to about 10 days, about 6 days to about 10 days, about 7 days to about 25 days, about 8 days to about 25 days, or about 9 days to about 12 days.

Genetically transduced T cells containing at least about 50% of CD28+ CD8+ T cells provided by methods described herein may exhibit at least about 1.2-fold higher, at least about 1.5-fold higher, at least about 2-fold higher, at least about 2.5-fold higher, at least about 3-fold higher, at least about 3.5-fold higher, at least about 4-fold higher, at least about 4.5-fold higher, or at least about 5-fold higher fold expansion than that produced from the determined population comprising less than about 50% of CD28+ CD8+ T cells.

Genetically transduced T cells containing at least about 50% of CD28+ CD8+ T cells provided by methods described herein may exhibit at least about 1.2-fold higher, at least about 1.5-fold higher, at least about 2-fold higher, at least about 2.5-fold higher, at least about 3-fold higher, at least about 3.5-fold higher, at least about 4-fold higher, at least about 4.5-fold higher, or at least about 5-fold higher ratio of CD8:CD4 T cells than that produced from the determined population comprising less than about 50% of CD28+ CD8+ T cells.

Genetically transduced T cells containing at least about 50% of CD28+ CD8+ T cells provided by methods described herein may exhibit at least about 1.2-fold longer, at least about 1.5-fold longer, at least about 2-fold longer, at least about 2.5-fold longer, at least about 3-fold longer, at least about 3.5-fold longer, at least about 4-fold longer, at least about 4.5-fold longer, or at least about 5-fold longer telomere length than that produced from the determined population comprising less than about 50% of CD28+ CD8+ T cells.

Genetically transduced T cells containing at least about 50% of CD28+ CD8+ T cells provided by methods described herein may exhibit at least about 1.2-fold higher, at least about 1.5-fold higher, at least about 2-fold higher, at least about 2.5-fold higher, at least about 3-fold higher, at least about 3.5-fold higher, at least about 4-fold higher, at least about 4.5-fold higher, or at least about 5-fold higher clonal richness than that produced from the determined population comprising less than about 50% of CD28+ CD8+ T cells.

Genetically transduced T cells produced by a method described herein may exhibit one or more of a higher fold expansion, a higher ratio of CD8:CD4 T cells, a longer telomere length, and/or a higher clonal richness as compared to those T cells T cells produced from a determined population containing less than about 50%, less than about 45%, less than about 40%, less than about 35%, less than about 30%, less than about 25%, less than about 20%, less than about 15%, less than about 10%, less than about 9%, less than about 8%, less than about 7%, less than about 6%, less than about 5%, less than about 4%, less than about 3%, less than about 2%, or less than about 1% of CD28+ CD8+ T cells.

Genetically transduced T cells selected from the determined population containing at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% of CD28+ CD8+ T cells may exhibit one or more of a higher fold expansion, a higher ratio of CD8:CD4 T cells, a longer telomere length, and/or a higher clonal richness as compared to those T cells produced from a determined population containing less than about 50%, less than about 45%, less than about 40%, less than about 35%, less than about 30%, less than about 25%, less than about 20%, less than about 15%, less than about 10%, less than about 9%, less than about 8%, less than about 7%, less than about 6%, less than about 5%, less than about 4%, less than about 3%, less than about 2%, or less than about 1% of CD28+ CD8+ T cells.

Described is a composition, for example a pharmaceutical composition, comprising the genetically transduced T cell obtainable by the herein described methods and a pharmaceutically acceptable carrier. Described are methods of treating a patient who has cancer, including administering to the patient an therapeutically effective amount of T cells produced by the method of any one of the afore-mentioned aspects, in which the cancer is selected from the group consisting of hepatocellular carcinoma (HCC), colorectal carcinoma (CRC), glioblastoma (GB), gastric cancer (GC), esophageal cancer, non-small cell lung cancer (NSCLC), pancreatic cancer (PC), renal cell carcinoma (RCC), benign prostate hyperplasia (BPH), prostate cancer (PCA), ovarian cancer (OC), melanoma, breast cancer, chronic lymphocytic leukemia (CLL), Merkel cell carcinoma (MCC), small cell lung cancer (SCLC), Non-Hodgkin lymphoma (NHL), acute myeloid leukemia (AML), gallbladder cancer and cholangiocarcinoma (GBC, CCC), urinary bladder cancer (UBC), acute lymphoblastic leukemia (ALL), multiple myeloma (MM), and uterine cancer (UEC).

Described is a composition, for example a pharmaceutical composition, comprising the genetically transduced T cells obtainable by the method of any one of the afore-mentioned aspects, for use as a medicament.

Described is a composition, for example a pharmaceutical composition, comprising the genetically transduced T cells obtainable by the method of any one of the afore-mentioned aspects, for use in the treatment of cancer, in which the cancer is selected from the group consisting of hepatocellular carcinoma (HCC), colorectal carcinoma (CRC), glioblastoma (GB), gastric cancer (GC), esophageal cancer, non-small cell lung cancer (NSCLC), pancreatic cancer (PC), renal cell carcinoma (RCC), benign prostate hyperplasia (BPH), prostate cancer (PCA), ovarian cancer (OC), melanoma, breast cancer, chronic lymphocytic leukemia (CLL), Merkel cell carcinoma (MCC), small cell lung cancer (SCLC), Non-Hodgkin lymphoma (NHL), acute myeloid leukemia (AML), gallbladder cancer and cholangiocarcinoma (GBC, CCC), urinary bladder cancer (UBC), acute lymphoblastic leukemia (ALL), multiple myeloma (MM), and uterine cancer (UEC).

Described is the use of a composition, for example a pharmaceutical composition, comprising the genetically transduced T cells obtainable by the method of any one of the afore-mentioned aspects, for the treatment of the cancer, in which the cancer is selected from the group consisting of hepatocellular carcinoma (HCC), colorectal carcinoma (CRC), glioblastoma (GB), gastric cancer (GC), esophageal cancer, non-small cell lung cancer (NSCLC), pancreatic cancer (PC), renal cell carcinoma (RCC), benign prostate hyperplasia (BPH), prostate cancer (PCA), ovarian cancer (OC), melanoma, breast cancer, chronic lymphocytic leukemia (CLL), Merkel cell carcinoma (MCC), small cell lung cancer (SCLC), Non-Hodgkin lymphoma (NHL), acute myeloid leukemia (AML), gallbladder cancer and cholangiocarcinoma (GBC, CCC), urinary bladder cancer (UBC), acute lymphoblastic leukemia (ALL), multiple myeloma (MM), and uterine cancer (UEC).

Described is a method of treating a patient who has cancer, including obtaining a population of CD8+ T cells from the patient, determining a % of CD28+ CD8+ T cells in the obtained population, activating the determined population with anti-CD3 antibody and anti-CD28 antibody, provided that the determined population comprises at least about 50% of CD28+ CD8+ T cells, or activating the determined population with anti-CD3 antibody in the absence of anti-CD28 antibody, provided that the determined population comprises less than about 50% of CD28+ CD8+ T cells, transducing the activated T cell population with a viral vector, expanding the transduced T cell population, and administering to the patient the expanded T cell population, in which the cancer is selected from the group consisting of hepatocellular carcinoma (HCC), colorectal carcinoma (CRC), glioblastoma (GB), gastric cancer (GC), esophageal cancer, non-small cell lung cancer (NSCLC), pancreatic cancer (PC), renal cell carcinoma (RCC), benign prostate hyperplasia (BPH), prostate cancer (PCA), ovarian cancer (OC), melanoma, breast cancer, chronic lymphocytic leukemia (CLL), Merkel cell carcinoma (MCC), small cell lung cancer (SCLC), Non-Hodgkin lymphoma (NHL), acute myeloid leukemia (AML), gallbladder cancer and cholangiocarcinoma (GBC, CCC), urinary bladder cancer (UBC), acute lymphoblastic leukemia (ALL), multiple myeloma (MM), and uterine cancer (UEC).

Described is a TCR binding to a peptide in a complex with a major histocompatibility complex (MHC) molecule, in which the peptide comprises the amino acid sequence selected from the group consisting of SEQ ID NO: 1-158.

In some embodiments of the methods of the invention, the viral vector may be a retroviral vector expressing a chimeric antigen receptor (CAR).

In some embodiments of the methods of the invention, the viral vector may be a lentiviral vector expressing a CAR.

In some embodiments of the methods of the invention, the CAR may be a CD19 CAR.

Described is a method of treating a patient who has cancer, comprising obtaining a population of CD8+ T cells from the patient, determining a % of CD28+ CD8+ T cells in the obtained population, activating the determined population with anti-CD3 antibody and anti-CD28 antibody, provided that the determined population comprises at least about 50% of CD28+ CD8+ T cells, or activating the determined population with anti-CD3 antibody in the absence of anti-CD28 antibody, provided that the determined population comprises less than about 50% of CD28+ CD8+ T cells, transducing the activated T cell population with a viral vector, expanding the transduced T cell population, determining a fold expansion of the expanded T cell population, administering to the patient the expanded T cell population, provided that the fold expansion is greater than 10-fold, wherein the cancer is selected from the group consisting of hepatocellular carcinoma (HCC), colorectal carcinoma (CRC), glioblastoma (GB), gastric cancer (GC), esophageal cancer, non-small cell lung cancer (NSCLC), pancreatic cancer (PC), renal cell carcinoma (RCC), benign prostate hyperplasia (BPH), prostate cancer (PCA), ovarian cancer (OC), melanoma, breast cancer, chronic lymphocytic leukemia (CLL), Merkel cell carcinoma (MCC), small cell lung cancer (SCLC), Non-Hodgkin lymphoma (NHL), acute myeloid leukemia (AML), gallbladder cancer and cholangiocarcinoma (GBC, CCC), urinary bladder cancer (UBC), acute lymphoblastic leukemia (ALL), multiple myeloma (MM), and uterine cancer (UEC).

The fold expansion may be about 2 to about 50 fold, about 5 to about 50 fold, about 10 to about 50, about 2 to about 30 fold, about 10 to about 20 fold, about 2 to about 25 fold, about 5 to about 25 fold, about 7 to about 20 fold, about 2 to about 10 fold, about 2 to about 5 fold. In another aspect, the fold expansion may be more than 2 fold, more than 3 fold, more than 4 fold, more than 5 fold, more than 8 fold, more than 10 fold, or more than 20 fold.

## BRIEF DESCRIPTION OF THE DRAWINGS

For a further understanding of the nature, objects, and advantages of the present disclosure, reference should be had to the following detailed description, read in conjunction with the following drawings, wherein like reference numerals denote like elements.

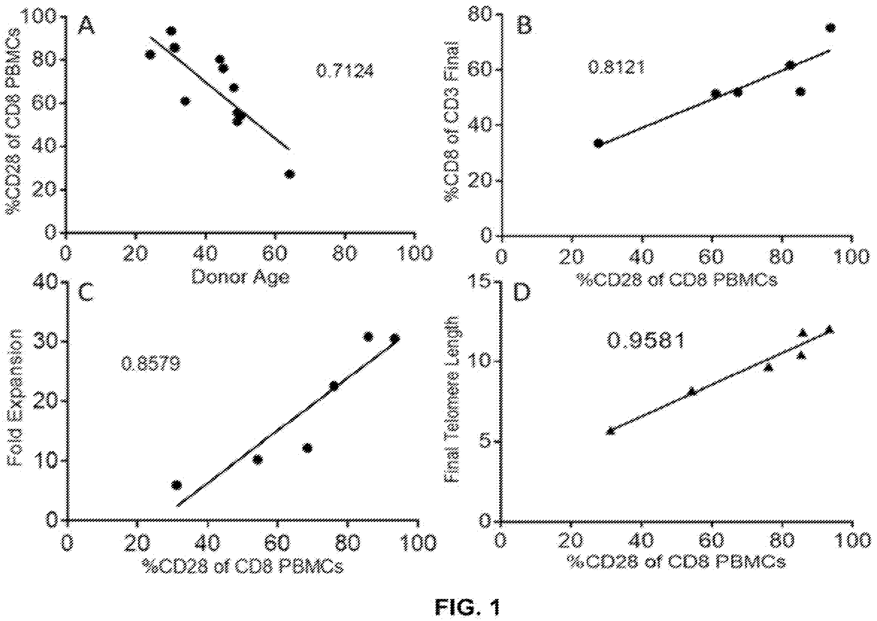

FIG. 1A shows the percentage of CD28 expression within the CD8 compartment of healthy human PBMCs across a large age gap in accordance with one embodiment of the present disclosure. Donors were analyzed by flow cytometry for CD28 expression. The linear correlation ($R^2 = 0.7124$), as determined by linear regression in Graphpad Prism 7, between starting CD28 expression in CD8 T-cells was observed.

FIG. 1B shows final percentage of CD8-positive cells within the CD3 compartment, i.e., CD8-positive and CD4-positive compartment, at the end of manufacturing for 7 days in accordance with one embodiment of the present disclosure. Starting CD28 percentage and final CD8 percentage were calculated by flow cytometry. There is an $R^2$ correlation of 0.8121, as determined by linear regression in Graphpad Prism 7, between the starting percentage of CD28 and the final CD8%.

FIG. 1C shows the fold expansion accomplished by 7 days in accordance with one embodiment of the present disclosure. Starting CD28 percentage was calculated by flow cytometry. Total fold expansion was calculated from the day of transduction to the day 7 in the culturing period. There is an $R^2$ correlation of 0.8579, as determined by linear regression in Graphpad Prism 7, between the starting percentage of CD28 and the final fold expansion.

FIG. 1D shows the final telomere length as measured by flow cytometry in accordance with one embodiment of the present disclosure. There is an $R^2$ correlation of 0.9581, as determined by linear regression in Graphpad Prism 7, between the starting percentage of CD28 and the final telomere length.

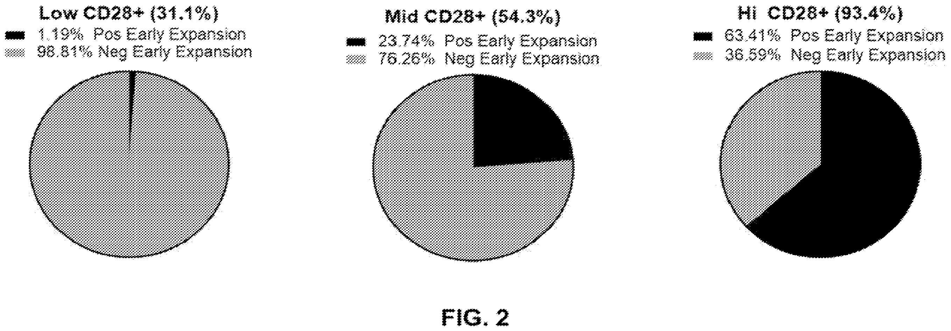

FIG. 2 shows characterization of T-cell expansion kinetics in accordance with one embodiment of the present disclosure. From 3 healthy donors, donor with higher (Hi), e.g., 93.4%, CD28 expression in the CD8 compartment of PBMCs contain more T-cell clones that can undergo an early expansion as defined by the cell number at day 4 vs the cell number at day 2 (2-day post activation with CD3/CD28) as compared with donors with medium (Mid), e.g., 54.3%, and low (Low), e.g., 31.1%, CD28 expression in the CD8 compartment of PBMCs.

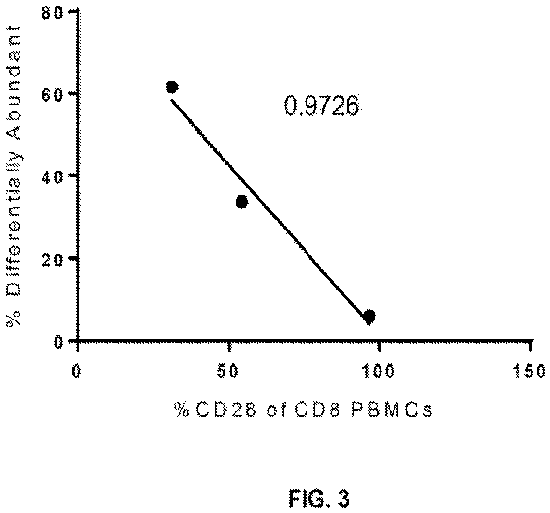

FIG. 3 shows contraction and expansion of clones correlate with starting CD28 percentage in accordance with one embodiment of the present disclosure. From 3 healthy donors, single molecule DNA sequencing was performed, and individual T-cell clones were tracked over time. The percent differentially abundant represents the fraction of all T-cell clones by day 10 in expansion that either expanded or contracted of the total number of evaluable T-cell clones relative to post-activation. Percentage of CD28 expressing cells was calculated by flow cytometry from the starting PBMCs. There is an $R^2$ correlation of 0.9726, as determined by linear regression in Graphpad Prism 7, between the starting percentage of CD28 and the percent differentially abundant.

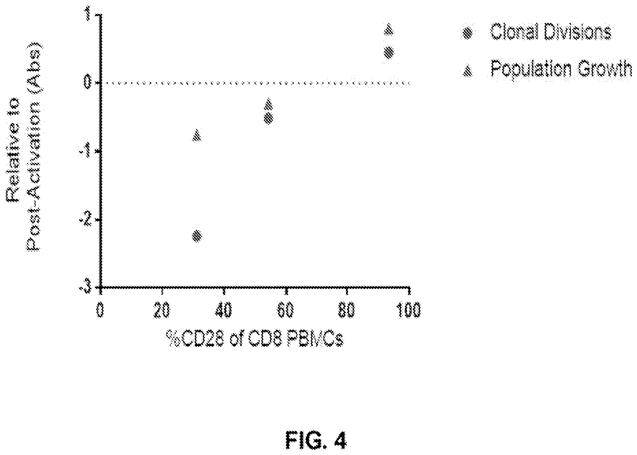

FIG. 4 shows low CD28 expressing donors exhibiting delayed T-cell expansion with negative clonal divisions in accordance with one embodiment of the present disclosure. Population growth may be calculated based on total viable cells and may represent fold growth. Clonal divisions were calculated as the log2(clonal fold expansion) and represent the median value obtained, negative values, i.e., below the dashed line, are obtained when clonal frequency contract in a culture, whereas positive values, i.e., above the dashed line, are obtained when clonal frequencies expand in a culture. All points are relative to the post-activation baseline and calculated to day 4 in the T-cell expansion process.

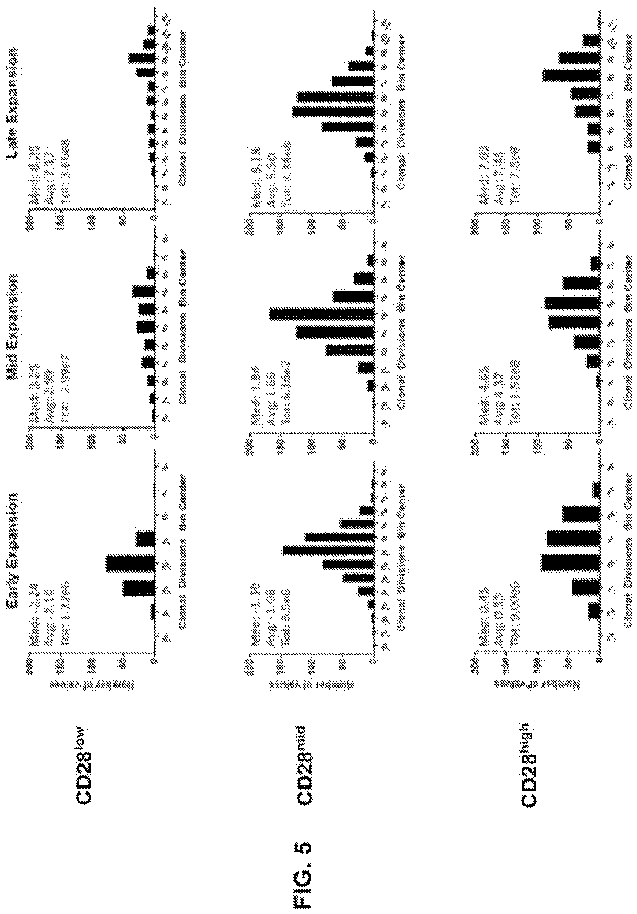

FIG. 5 shows characterization of T-cell expansion kinetics in accordance with another embodiment of the present disclosure. T-cell clones were binned based on the number of divisions they had undergone, estimated by $\log_2$(fold growth) for each T-cell clone. Early, mid, and late expansion correspond to day 4, 7, and 10 in the manufacturing process. Inserts contain the median (Med) and average (Avg) clonal division along with the total (Tot) number of cells at the time.

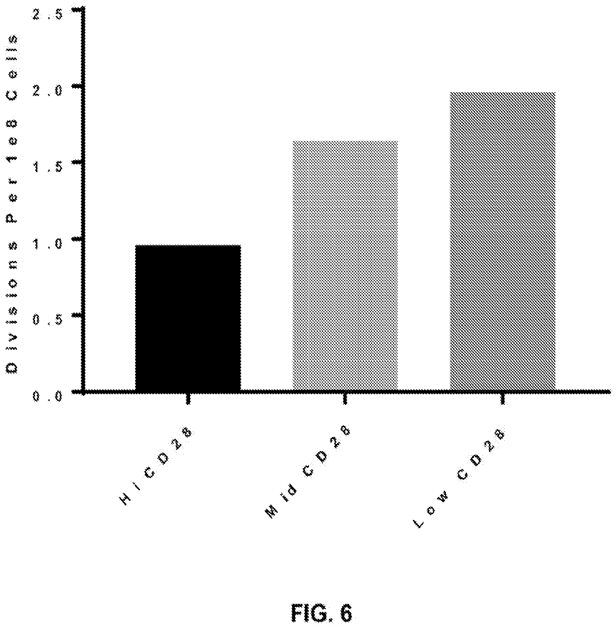

FIG. 6 shows characterization of T-cell expansion kinetics in accordance with another embodiment of the present disclosure. The number of divisions required to reach 100 million cells was calculated based on the average divisions by the late expansion timepoint.

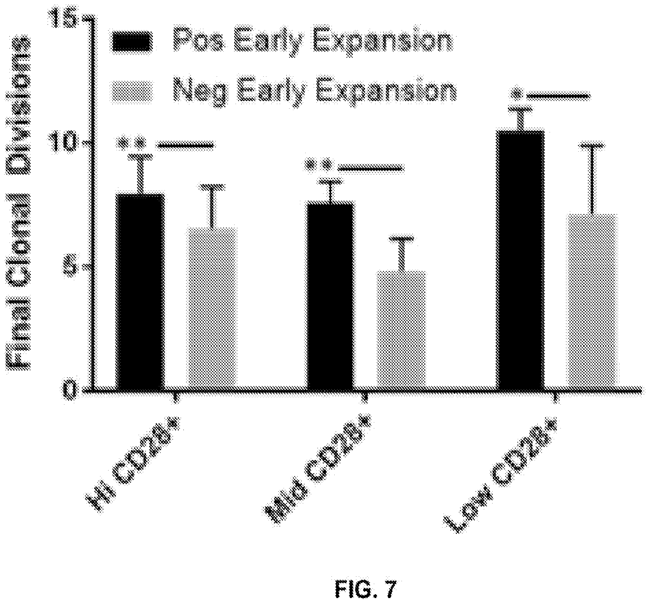

FIG. 7 shows characterization of T-cell expansion kinetics in accordance with another embodiment of the present disclosure. The average final clonal divisions between T-cell clones that underwent a positive or negative early expansion (day 2 to day 4) were calculated. *P<0.05, **P<.0001.

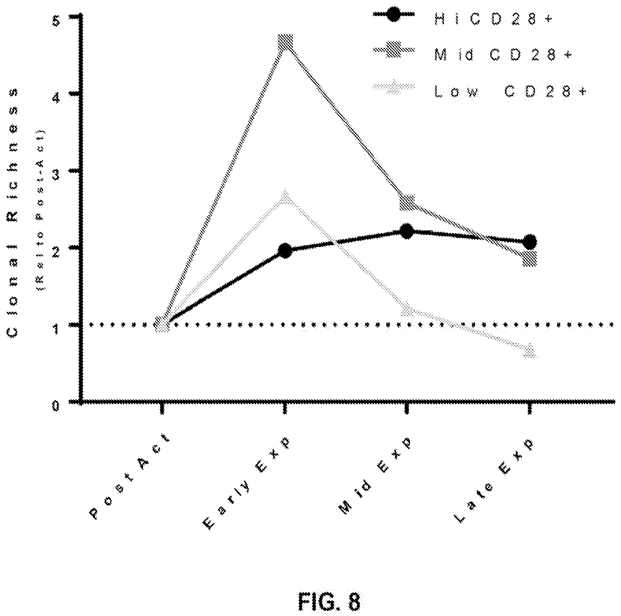

FIG. 8 shows characterization of T-cell expansion kinetics in accordance with another embodiment of the present disclosure. Following the burst in unique clones after stimulation, there is a continual reduction in unique clones in donors with lower CD28, e.g., Mid CD28+ and Low CD28+. Unique T-cell clones may be derived from the number of unique DNA molecule reads of the T-cell receptor (TCR) CDR3 region. Dotted line at value of 1 marks the point where there are fewer T-cell clones than existed post-activation. Clonal diversity (number of unique clones) was measured across the T-cell manufacturing procedure at early (day 4), mid (day 7), and late (day 10). All values are normalized to the number of unique T-cell clones at post-activation (day 2) timepoint.

## DETAILED DESCRIPTION

Adoptive T-cell therapy using genetically modified T cells has emerged as a potential therapeutic option for several malignancies. Central to the production of the cellular therapy is the manufacturing using a combination of stimulation, genetic engineering, and expansion methodology. Within this framework, there may be a delicate balance between expansion of the cells to a therapeutically relevant dosage and the need to retain the proliferative potential of the "living drug."

As described herein, the disclosure provides for methods of improving the efficacy of T cells and for methods of enhancing and predicting final fold expansion, ratio of CD8:CD4 T cells, the relative final telomere length, and clonal richness of the T-cell product. The disclosure also provides for methods of treating cancer in a subject in need thereof as well as T cells populations produced by methods described herein.

CD28 is one of the molecules expressed on T cells that provide co-stimulatory signals, which are required for T cell activation. CD28 is the receptor for B7.1 (CD80) and B7.2 (CD86). When activated by Toll-like receptor ligands, the B7.1 expression is upregulated in antigen presenting cells (APCs). The B7.2 expression on antigen presenting cells is constitutive. CD28 is the only B7 receptor constitutively expressed on naive T cells. Stimulation through CD28 in addition to the TCR can provide a potent co-stimulatory signal to T cells for the production of various interleukins (IL-2 and IL-6 in particular).

When T-cells were expanded for elongated periods of time, they may lose their proliferative potential and become functionally senescent despite the presence of multiple proliferative cytokines. In addition, expression of CD28 may correlate with multiple manufacturing metrics, including final T-cell fold expansion. Thus, the loss of CD28 expression may create a T-cell expansion bottleneck, in which certain T-cell clones may be heavily favored as compared to others during manufacturing. Compounding the multiple correlations, meta-analysis of available clinical trial data shows that younger patients appear to respond better to T-cell manufacturing involving CD28 costimulation, while older patients appear to respond better to T-cell manufacturing lacking CD28 costimulation.

In an aspect of the present disclosure, the starting percentage of CD28-positive CD8+ T cells may be used as a biomarker to enable accurate prediction of 1) fold T-cell expansion, 2) ratio of CD8:CD4 T-cells (or %CD8-positive cells of CD3-positive cells), and 3) relative telomere length of the final T-cell product. Additionally, CDR3 DNA sequencing may be used to track clonal populations from donors with varying starting CD28 expression levels. From this analysis, different CD28 starting expression levels may result in significant differences in clonal expansion kinetics throughout the T-cell manufacturing process.

The process of T-cell manufacturing relies on the isolation, activation, and expansion of PBMC derived T-cells. The activation may be accomplished via immobilized agonistic antibodies against CD3 and CD28 followed by the expansion in a cytokine milieu. During manufacturing, product characteristics, such as fold T-cell expansion and the ratio of CD8+ to CD4+ cells, may be tracked as they may impact therapeutic efficacy and meet minimal thresholds. Therefore, it may be desirable to have a deeper knowledge of the factors that can influence these metrics and affect the outcome of clinical manufacturing.

For example, the process of making the T-cell product may be generally divided into five steps: (1) leukapheresis to isolate the patients peripheral blood mononuclear cells (PBMCs), (2) activation, (3) genetic modification of the T cells from the PBMCs with a non-viral or virally encoded TCR/CAR vector, (4) expansion of the T cells to create a clinically relevant dose, and (5) optional lymphodepletion of the patient before T-cell infusion, and infusion of the modified T cells into the patient. The activation of the T-cell compartment may be primarily achieved via the use of agonistic $\alpha$CD3 antibody with or without costimulatory stimulation via $\alpha$CD28 antibody, followed by the expansion in, usually, IL-2, though IL-7 + IL-15 may yield a naive T-cell final product.

During the expansion process, T cells may be in balance between growth and contraction due to TCR stimulation withdrawal. During manufacturing, T-cells differentiate towards terminally-differentiated effector cells, and this process may be dependent on the starting differentiation status of the PBMC. PBMCs from older donors may be enriched for CD28-negative CD8+ T cells. Additionally, non-apoptotic extrinsic Fas-based T cell-T cell interactions may drive differentiation of naive T cells. These observations indicate that certain T cells may outcompete others during T-cell ex vivo expansion. Thus, the dynamics of this contraction and expansion may need to be elucidated at a clonal level.

In certain aspects, the T cells of the present disclosure may include primary human T cells, such as T cells derived from human peripheral blood mononuclear cells (PBMC), PBMC collected after stimulation with G-CSF, bone marrow, or umbilical cord blood. Conditions may include the use of mRNA and DNA and electroporation. Following transfection, cells may be immediately infused or may be stored. In certain aspects, following transfection, the cells may be propagated for days, weeks, or months ex vivo as a bulk population within about 1, about 2, about 3, about 4, or about 5 days or more following gene transfer into cells.

In a further aspect, following transfection, the transfectants may be cloned and a clone demonstrating presence of a single integrated or episomally maintained expression cassette or plasmid, and expression of the TCR may be expanded ex vivo. The clone selected for expansion may demonstrate the capacity to specifically recognize and lyse peptide-expressing target cells. The recombinant T cells may be expanded by stimulation with IL-2, or other cytokines that bind the common gamma-chain (e.g., IL-7, IL-10, IL-12, IL-15, IL-21, and others). The recombinant T cells may be expanded by stimulation with artificial antigen presenting cells. The recombinant T cells may be expanded on artificial antigen presenting cell or with an antibody, such as OKT3, which cross links CD3 on the T cell surface. Subsets of the recombinant T cells may be deleted on artificial antigen presenting cell or with an antibody, such as Campath, which binds CD52 on the T cell surface. In a further aspect, the genetically modified cells may be cryopreserved.

The term "activation" refers to the state of a T cell that has been sufficiently stimulated to induce detectable cellular proliferation. In particular embodiments, activation can also be associated with induced cytokine production, and detectable effector functions. The term "activated T cells" refers to, among other things, T cells that are proliferating. Signals generated through the TCR alone are insufficient for full activation of the T cell and one or more secondary or costimulatory signals are also required. Thus, T cell activation comprises a primary stimulation signal through the TCR/CD3 complex and one or more secondary costimulatory signals. Co-stimulation can be evidenced by proliferation and/or cytokine production by T cells that have received a primary activation signal, such as stimulation through the CD3/TCR complex or through CD2.

Disclosed is a method of making and/or expanding the antigen-specific redirected T cells that comprises transfecting T cells with an expression vector containing a DNA construct encoding TCR, then, optionally, stimulating the cells with antigen positive cells, recombinant antigen, or an antibody to the receptor to cause the cells to proliferate.

Disclosed is a method of stably transfecting and re-directing T cells by electroporation, or other non-viral gene transfer (such as, but not limited to sonoporation) using naked DNA or RNA. Most investigators have used viral vectors to carry heterologous genes into T cells. By using naked DNA or RNA, the time required to produce redirected T cells can be reduced. "Naked DNA or RNA" means DNA or RNA encoding a TCR contained in an expression cassette or vector in proper orientation for expression. The electroporation method of this disclosure produces stable transfectants that express and carry on their surfaces the TCR.

In certain aspects, TCR construct may be introduced into the subject's own T cells as naked DNA or in a suitable vector. Methods of stably transfecting T cells by electroporation using naked DNA in the art. See, e.g., U.S. Pat. No. 6,410,319 Naked DNA generally refers to the DNA encoding a TCR of the present disclosure contained in a plasmid expression vector in proper orientation for expression. Advantageously, the use of naked DNA reduces the time required to produce T cells expressing the TCR of the present disclosure.

Alternatively, a viral vector (e.g., a retroviral vector, adenoviral vector, adenoassociated viral vector, or lentiviral vector) can be used to introduce the TCR construct into T cells. Suitable vectors for use in accordance with the method of the present disclosure are non-replicating in the subject's T cells. A large number of vectors are known that are based on viruses, where the copy number of the virus maintained in the cell is low enough to maintain the viability of the cell. Illustrative vectors include the pFB-neo vectors (STRATAGENE®) as well as vectors based on HIV, SV40, EBV, HSV, or BPV.

Once it is established that the transfected or transduced T cell is capable of expressing the TCR construct as a surface membrane protein with the desired regulation and at a desired level, it can be determined whether the TCR is functional in the host cell to provide for the desired signal induction. Subsequently, the transduced T cells are reintroduced or administered to the subject to activate anti-tumor responses in the subject.

To facilitate administration, the transduced T cells according to the disclosure can be made into a pharmaceutical composition or made into an implant appropriate for administration in vivo, with appropriate carriers or diluents, which further can be pharmaceutically acceptable. The means of making such a composition or an implant have been described in the art (see, for instance, Remington's Pharmaceutical Sciences, 16th Ed., Mack, ed. (1980)). Where appropriate, the transduced T cells can be formulated into a preparation in semisolid or liquid form, such as a capsule, solution, injection, inhalant, or aerosol, in the usual ways for their respective route of administration. Means known in the art can be utilized to prevent or minimize release and absorption of the composition until it reaches the target tissue or organ, or to ensure timed-release of the composition. Desirably, however, a pharmaceutically acceptable form is employed that does not hinder the cells from expressing the TCR. Thus, desirably the transduced T cells can be made into a pharmaceutical composition containing a balanced salt solution, preferably Hanks' balanced salt solution, or normal saline.

The method of the present disclosure can be used to expand selected T cell populations for use in treating an infectious disease or cancer. The resulting T cell population can be genetically transduced and used for immunotherapy or can be used for in vitro analysis of infectious agents. Following expansion of the T cell population to sufficient numbers, the expanded T cells may be restored to the individual. The method of the present disclosure may also provide a renewable source of T cells. Thus, T cells from an individual can be expanded ex vivo, a portion of the expanded population can be readministered to the individual and another portion can be frozen in aliquots for long term preservation, and subsequent expansion and administration to the individual. Similarly, a population of tumor-infiltrating lymphocytes can be obtained from an individual afflicted with cancer and the T cells stimulated to proliferate to sufficient numbers and restored to the individual.

In an aspect, expansion and/or activation of T cells take place in the presence of one or more of IL-2, IL-7, IL-10, IL-12, IL-15, IL-21. In another aspect, expansion and/or activation of T cells takes place with IL-2 alone, IL-7 alone, IL-15 alone, a combination of IL-2 and IL-15, or a combination of IL-7 and IL-15.

Disclosed are compositions containing an agent that provides a costimulatory signal to a T cell for T cell expansion (e.g., an anti-CD28 antibody, B7-1 or B7-2 ligand), coupled to a solid phase surface, which may additionally include an agent that provides a primary activation signal to the T cell (e.g., an anti-CD3 antibody) coupled to the same solid phase surface. These agents may be preferably attached to beads or flasks or bags. Compositions comprising each agent coupled to different solid phase surfaces (i.e., an agent that provides a primary T cell activation signal coupled to a first solid phase surface and an agent that provides a costimulatory signal coupled to a second solid phase surface) may also be within the scope of this disclosure.

The disclosed composition can be provided in unit dosage form, in which each dosage unit, e.g., an injection, may contain a predetermined amount of the composition, alone or in appropriate combination with other active agents. The term unit dosage form as used herein refers to physically discrete units suitable as unitary dosages for human and animal subjects, each unit containing a predetermined quantity of the composition of the present invention, alone or in combination with other active agents, calculated in an amount sufficient to produce the desired effect, in association with a pharmaceutically acceptable diluent, carrier, or vehicle, where appropriate. The specifications for the novel unit dosage forms of the present disclosure depend on the particular pharmacodynamics associated with the pharmaceutical composition in the particular subject.

Desirably, an effective amount or sufficient number of the isolated transduced T cells is present in the composition and introduced into the subject such that long-term, specific, anti-tumor responses may be established to reduce the size of a tumor or eliminate tumor growth or regrowth than would otherwise result in the absence of such treatment. Desirably, the amount of transduced T cells reintroduced into the subject may cause about 10%, about 20%, about 30%, about 40%, about 50%, about 60%, about 70%, about 80%, about 90%, about 95%, about 98%, or about 99% decrease in tumor size when compared to otherwise same conditions, in which the transduced T cells are not present.

Accordingly, the amount of transduced T cells administered should take into account the route of administration and should be such that a sufficient number of the transduced T cells will be introduced so as to achieve the desired therapeutic response. Furthermore, the amounts of each active agent included in the compositions described herein (e.g., the amount per each cell to be contacted or the amount per certain body weight) can vary in different applications. In general, the concentration of transduced T cells desirably should be sufficient to provide in the subject being treated at least from about $1 \times 10^6$ to about $1 \times 10^9$ transduced T cells/m$^2$ (or kg) of a patient, even more desirably, from about $1 \times 10^7$ to about $5 \times 10^8$ transduced T cells/m$^2$ (or kg) of a patient, although any suitable amount can be utilized either above, e.g., greater than $5 \times 10^8$ cells/m$^2$ (or kg) of a patient, or below, e.g., less than $1 \times 10^7$ cells/m$^2$ (or kg) of a patient. The dosing schedule can be based on well-established cell-based therapies (see, e.g., U.S. Pat. No. 4,690,915), or an alternate continuous infusion strategy can be employed.

These values may provide general guidance of the range of transduced T cells to be utilized by the practitioner upon optimizing the method of the present disclosure for practice of the invention. The recitation herein of such ranges by no means precludes the use of a higher or lower amount of a component, as might be warranted in a particular application. For example, the actual dose and schedule can vary depending on whether the compositions are administered in combination with other pharmaceutical compositions, or depending on interindividual differences in pharmacokinetics, drug disposition, and metabolism. One skilled in the art readily can make any necessary adjustments in accordance with the exigencies of the particular situation.

In an aspect, tumor associated antigen (TAA) peptides that are capable of use with the methods and embodiments described herein include, for example, those TAA peptides described in U.S. Publication 20160187351, U.S. Publication 20170165335, U.S. Publication 20170035807, U.S. Publication 20160280759, U.S. Publication 20160287687, U.S. Publication 20160346371, U.S. Publication 20160368965, U.S. Publication 20170022251, U.S. Publication 20170002055, U.S. Publication 20170029486, U.S. Publication 20170037089, U.S. Publication 20170136108, U.S. Publication 20170101473, U.S. Publication 20170096461, U.S. Publication 20170165337, U.S. Publication 20170189505, U.S. Publication 20170173132, U.S. Publication 20170296640, U.S. Publication 20170253633, U.S. Publication 20170260249, U.S. Publication 20180051080, and U.S. Publication No. 20180164315.

In an aspect, T cells described herein selectively recognize cells which present a TAA peptide described in one of more of the patents and publications described above.

In another aspect, TAA that are capable of use with the methods and embodiments described herein include at least one selected from SEQ ID NO: 1 to SEQ ID NO: 158. In an aspect, T cells selectively recognize cells which present a TAA peptide described in SEQ ID NO: 1 - 158 or any of the patents or applications described herein.

EP 3 941 487 B1

| SEQ ID NO: | Amino Acid Sequence | SEQ ID NO: | Amino Acid Sequence | SEQ ID NO: | Amino Acid Sequence |
|---|---|---|---|---|---|
| 1 | YLYDSETKNA | 54 | LLWGHPRVALA | 106 | VLLNEILEQV |
| 2 | HLMDQPLSV | 55 | VLDGKVAVV | 107 | SLLNQPKAV |
| 3 | GLLKKINSV | 56 | GLLGKVTSV | 108 | KMSELQTYV |
| 4 | FLVDGSSAL | 57 | KMISAIPTL | 109 | ALLEQTGDMSL |
| 5 | FLFDGSANLV | 58 | GLLETTGLLAT | 110 | VIIKGLEEITV |
| 6 | FLYKIIDEL | 59 | TLNTLDINL | 111 | KQFEGTVEI |
| 7 | FILDSAETTTL | 60 | VIIKGLEEI | 112 | KLQEEIPVL |
| 8 | SVDVSPPKV | 61 | YLEDGFAYV | 113 | GLAEFQENV |
| 9 | VADKIHSV | 62 | KIWEELSVLEV | 114 | NVAEIVIHI |
| 10 | IVDDLTINL | 63 | LLIPFTIFM | 115 | ALAGIVTNV |
| 11 | GLLEELVTV | 64 | ISLDEVAVSL | 116 | NLLIDDKGTIKL |
| 12 | TLDGAAVNQV | 65 | KISDFGLATV | 117 | VLMQDSRLYL |
| 13 | SVLEKEIYSI | 66 | KLIGNIHGNEV | 118 | KVLEHVVRV |
| 14 | LLDPKTIFL | 67 | ILLSVLHQL | 119 | LLWGNLPEI |
| 15 | YTFSGDVQL | 68 | LDSEALLTL | 120 | SLMEKNQSL |
| 16 | YLMDDFSSL | 69 | VLQENSSDYQSNL | 121 | KLLAVIHEL |
| 17 | KVWSDVTPL | 70 | HLLGEGAFAQV | 122 | ALGDKFLLRV |
| 18 | LLWGHPRVALA | 71 | SLVENIHVL | 123 | FLMKNSDLYGA |
| 19 | KIWEELSVLEV | 72 | YTFSGDVQL | 124 | KLIDHQGLYL |
| 20 | LLIPFTIFM | 73 | SLSEKSPEV | 125 | GPGIFPPPPPQP |
| 21 | FLIENLLAA | 74 | AMFPDTIPRV | 126 | ALNESLVEC |
| 22 | LLWGHPRVALA | 75 | FLIENLLAA | 127 | GLAALAVHL |
| 23 | FLLEREQLL | 76 | FTAEFLEKV | 128 | LLLEAVWHL |
| 24 | SLAETIFIV | 77 | ALYGNVQQV | 129 | SIIEYLPTL |
| 25 | TLLEGISRA | 78 | LFQSRIAGV | 130 | TLHDQVHLL |
| 26 | ILQDGQFLV | 79 | ILAEEPIYIRV | 131 | SLLMWITQC |
| 27 | VIFEGEPMYL | 80 | FLLEREQLL | 132 | FLLDKPQDLSI |
| 28 | SLFESLEYL | 81 | LLLPLELSLA | 133 | YLLDMPLWYL |
| 29 | SLLNQPKAV | 82 | SLAETIFIV | 134 | GLLDCPIFL |
| 30 | GLAEFQENV | 83 | AILNVDEKNQV | 135 | VLIEYNFSI |
| 31 | KLLAVIHEL | 84 | RLFEEVLGV | 136 | TLYNPERTITV |
| 32 | TLHDQVHLL | 85 | YLDEVAFML | 137 | AVPPPPSSV |
| 33 | TLYNPERTITV | 86 | KLIDEDEPLFL | 138 | KLQEELNKV |
| 34 | KLQEKIQEL | 87 | KLFEKSTGL | 139 | KLMDPGSLPPL |
| 35 | SVLEKEIYSI | 88 | SLLEVNEASSV | 140 | ALIVSLPYL |
| 36 | RVIDDSLVVGV | 89 | GVYDGREHTV | 141 | FLLDGSANV |
| 37 | VLFGELPAL | 90 | GLYPVTLVGV | 142 | ALDPSGNQLI |
| 38 | GLVDIMVHL | 91 | ALLSSVAEA | 143 | ILIKHLVKV |

11

(continued)

| SEQ ID NO: | Amino Acid Sequence | SEQ ID NO: | Amino Acid Sequence | SEQ ID NO: | Amino Acid Sequence |
|---|---|---|---|---|---|
| 39 | FLNAIETAL | 92 | TLLEGISRA | 144 | VLLDTILQL |
| 40 | ALLQALMEL | 93 | SLIEESEEL | 145 | HLIAEIHTA |
| 41 | ALSSSQAEV | 94 | ALYVQAPTV | 146 | SMNGGVFAV |
| 42 | SLITGQDLLSV | 95 | KLIYKDLVSV | 147 | MLAEKLLQA |
| 43 | QLIEKNWLL | 96 | ILQDGQFLV | 148 | YMLDIFHEV |
| 44 | LLDPKTIFL | 97 | SLLDYEVSI | 149 | ALWLPTDSATV |
| 45 | RLHDENILL | 98 | LLGDSSFFL | 150 | GLASRILDA |
| 46 | YTFSGDVQL | 99 | VIFEGEPMYL | 151 | ALSVLRLAL |
| 47 | GLPSATTTV | 100 | ALSYILPYL | 152 | SYVKVLHHL |
| 48 | GLLPSAESIKL | 101 | FLFVDPELV | 153 | VYLPKIPSW |
| 49 | KTASINQNV | 102 | SEWGSPHAAVP | 154 | NYEDHFPLL |
| 50 | SLLQHLIGL | 103 | ALSELERVL | 155 | VYIAELEKI |
| 51 | YLMDDFSSL | 104 | SLFESLEYL | 156 | VHFEDTGKTLLF |
| 52 | LMYPYIYHV | 105 | KVLEYVIKV | 157 | VLSPFILTL |
| 53 | KVWSDVTPL | | | 158 | HLLEGSVGV |

In an aspect, T cell receptors capable of use with methods described herein, include, for example, those described in U.S. Publication No. 20170267738, U.S. Publication No. 20170312350, U.S. Publication No. 20180051080, U.S. Publication No. 20180164315, U.S. Publication No. 20180161396, U.S. Publication No. 20180162922, U.S. Publication No. 20180273602, U.S. Publication No. 20190002556, U.S. Publication NO. 20180135039,.

The genetically transduced T cells produced by a method described herein have an improved efficacy, more particularly an improved efficacy for immunotherapy, such as adoptive immunotherapy, since, as it will be understood by the skilled in the art, the genetically transduced T cells produced by a method described herein exhibit one or more of a higher fold expansion, a higher ratio of CD8:CD4 T cells, a longer telomere length, and/or a higher clonal richness as compared to those T cells T cells produced from a determined population containing less than about 50%, less than about 45%, less than about 40%, less than about 35%, less than about 30%, less than about 25%, less than about 20%, less than about 15%, less than about 10%, less than about 9%, less than about 8%, less than about 7%, less than about 6%, less than about 5%, less than about 4%, less than about 3%, less than about 2%, or less than about 1% of CD28+ CD8+ T cells.

**EXAMPLES:**

In the design of T-cell manufacturing protocols, a balance may exist between the need to expand T cells to meet desired cell numbers and retaining the proliferative potential of the final T-cell product. Within this paradigm, there may be a benefit to predicting the outcome of manufacturing based on attributes of the starting PBMC population.

The heterogeneity in T-cell expression profiles observed suggests that necessary criteria for a selective pressure may exist. This pressure, in theory, may potentially limit the replicative potential of specific T cell populations. From the analysis of costimulatory molecules, there may be a loss of CD28 expression in the starting CD8 cells and throughout the T-cell expansion protocol. Reasonably, this would create two types of CD8 T cells, those which would benefit from the CD28 co-stimulation given and those that would not benefit. The following examples illustrates an intrinsic CD28 importance and the correlations between the starting CD28 phenotype and multiple manufacturing metrics.

Example 1

*T-Cell Manufacturing*

Healthy donor whole blood was purchased from Hemacare and PBMCs were isolated by Ficoll gradient. PBMCs were activated for 16-24 hours in TexMACS (Miltenyi 130-097-196) supplemented with 5% Human AB serum (Gemini 100-318) media by plating at 1x $10^6$ live PBMC/mL on tissue culture flasks coated overnight with 1 ug/mL anti-CD3 (eBioscience 16-0037-85) and 1 ug/mL anti-CD28 (eBioscience 16-0289-85) antibody in PBS (Lonza 17-516F) at 4 degrees Celsius. The next day, total cells were isolated and resuspended to 1x $10^6$ live-cell/ml and 5 ml were plated into a well of a Grex24 well plate (Wilson Wolf 80192M). Cells were either mock transduced or transduced with a TCR lentiviral construct (produced by Lentigen) in the presence of 10 ng/ml IL-7 (peprotech 200-07), 100 ng/ml IL-15 (peprotech 200-15), and 10 $\mu$g/ml protamine sulfate. The next day, cells were fed with 35 ml of complete TexMACS supplemented with IL-7 and IL-15 at above mentioned concentrations. Cells were grown for an additional 2, 5, or 8 days depending on the desired manufacturing time (i.e., 4, 7, or 10 total days). After manufacturing, cells were counted and frozen down at $5 \times 10^6$/ml in Cyrostore10, placed at -80 degrees Celsius for 16-24 hours and then stored long-term at LN2 vapor phase until needed.

*PkH67 stain*

PkH67 (Sigma PKH67GL) stain was performed per manufacturer's protocol with the exception that the day 4 manufactured cells were stained at a 2X concentration to account for the larger cell size compared to day 7 or day 10 manufactured cells. PkH staining was performed before the flow cytometry viability dye stain.

*CDR3 sequencing (Adaptive Biotech) and Analysis of T-cell receptor variable beta chain sequencing*

Immunosequencing of the CDR3 regions of human TCRβ chains was performed using the immunoSEQ® Assay (Adaptive Biotechnologies, Seattle, WA). Extracted genomic DNA was amplified in a bias-controlled multiplex PCR, followed by highthroughput sequencing. Sequences were collapsed and filtered in order to identify and quantitate the absolute abundance of each unique TCRβ CDR3 region for further analysis.

*Flow Cytometry Stain and Acquisition*

Live cells were quantified and resuspended to 1-2 $\times 10^6$ live-cell/ml in PBS then stained with Live-Dead aqua (Thermo Fisher L34957) stain according to manufacturer's protocol. Cells were then washed with Flow buffer and then resuspended at desired antibody mix (CD3 PerCp-Cy5.5 Biolegend 300328, Vb8 PE Biolegend, 348104, CD45Ro PE-Cy7 Biolegend 304230, CD95 APC-fire750 Biolegend 305638, CD8 BV605 BD 564116, CD27 BV650 Biolegend 302827, CD62L BV785 Biolegend 304830) and stained for 15-30 minutes in the dark at 4 degrees Celsius, with the exception that the CCR7 (CCR7 BV41 Biolegend 353208) stain was done at 37 degrees Celsius in RPMI without serum before the remaining surface stains. Cells were then washed in Flow buffer and resuspended in fixation buffer and stored at 4 degrees Celsius until acquired on the BD Fortessa or Miltenyi MACSQuant analyzer.

*Telomere length determination*

Relative telomere length was determined according to manufacturer's instructions (Dako/Agilent K5327). Briefly, T-cells were mixed at a 1:1 ratio with control 1301 tumor cells (4N genome). Cells were then permeabilized and a Telomere PNA FITC probe was hybridized overnight. The next day, a counter propidium iodide stain was performed to discriminate intact cells and the cells were acquired by flow cytometry. The telomere length of the test cells was calculated as a ratio to that of the control 1301 tumor cell line.

Example 2

*CD28 expression on CD8+ T-cells serves* as a *biomarker for ex vivo T-cell expansion with IL-7 and IL-15*

*The age correlated loss of CD28 in CD8 T cells*

For a selective pressure between donors, there may be an intrinsic heterogeneity between donors. The manufacturing of a T-cell product from PBMC relies on the ability to efficiently activate and expand antigen-specific cytolytic CD8 T cells. During this process, there may be a need to track the growth of the cells as minimal dosages. This need may often be met based on the design of the clinical trial. Manufacturing of T cell products from elderly PBMC can be complicated by the accumulation of CD28-negative CD8+ T cells in the blood.

FIG. 1A shows, from the CD28 profiling, the older the donor was, the lower the starting percentage of CD8 cells that expressed CD28, with an $R^2$ correlation of 0.7124, as determined by linear regression in Graphpad Prism 7.

These cells may have reduced proliferative potential to both cognate peptide and stimulation via CD3/CD28.

IL-7 and IL-15 may preserve T-cell naivety as compared to use of IL-2 during T-cell expansion. As such, IL-7 and IL-15 may be a preferred method for clinical manufacturing. Additionally, CD28-negative CD8+ T cells may proliferate in response to IL15 comparably to their CD28-positive counterparts. To compare how CD28 expression would affect the manufacturing of PBMC derived T-cells in the presence of IL-7 and IL-15, T-cells obtained from 6 healthy donors were manufactured using a clinical-like process.

*CD28 starting percentage correlates with final CD8 percentage during T-cell expansions*

Since CD28 expression in the CD8 compartment may be age correlated, other manufacturing metrics, which depend on CD28 expression, may also be biased. At the end of T-cell expansion, the ratio of CD8 to CD4 cells (or %CD8-positive cells of CD3-positive cells) may be measured as it is primarily the CD8 compartment that performs tumor cytolytic function, though cytolytic CD4 cells have been identified. Thus, there may be a correlation between the starting CD28 expression in the CD8 cells and the final percentage of cells.

FIG. 1B shows there is a correlation between the starting percentage of CD28 expression in the CD8+ T-cell compartment and the final %CD8-positive cells of CD3-positive cells at day 7 (mid expansion) of the culture with an $R^2$ correlation of 0.8121. These results suggest that CD28 expression may serves as the driving force for the selective pressure for CD8+ T cells.

*CD28 starting percentage correlates with fold expansion*

Clinical T-cell expansion protocols often measure the fold expansion of the final product as a metric to understand the number of population doublings that have taken place.

FIG. 1C shows, by day 7 (mid expansion), in the expansion protocol, there was a clear correlation between fold expansion and the starting CD28 expression level with an $R^2$ correlation of 0.8579. The outgrowth of CD8+ cells compared to CD4+ cells correlates tightly with the starting percentage of CD28 expression on CD8+ T cells. These results have implications for manufacturing process development as it can predict whether clinical expansion would be successful based on the starting phenotype of the PBMCs.

*Telomere length reduction correlates with the starting CD28 expression*

The loss of telomere length is a hallmark of dysfunctional cells as they become highly differentiated and eventually senescent. The expression of telomerase may be restricted to the CD28 expressing cells of either the CD4 or CD8 compartment following CD3 + CD28 stimulation. Thus, the final relative telomere length may also correspond with this CD28 expressing fraction of cells.

FIG. 1D shows the final relative telomere length of the T-cell product may be closely correlated with the level of CD28 expression on CD8+ T cells in the starting culture with an $R^2$ correlation of 0.9581 between the starting CD28 percentage of cells in the PBMCs and the final relative telomere length. This analysis was carried out from PBMCs derived from multiple healthy donors and multiple non-small cell lung cancer patients. This data suggests that the outcome of IL-7/IL-15 based T-cell manufacturing can be predicted prior to culture initiation and may have important implications for the design of adoptive T-cell manufacturing protocols. For example, because persistence of infused cell therapy products may be correlated with clinical outcome in cancer patients, the final telomere length of infused tumor-infiltrating lymphocyte (TIL) clinical products may be associated with the persistence of T-cell clones.

Taken together, by measuring the CD28 expression of the starting CD8+ T-cells, one can reasonably predict the final CD8%, the fold expansion, and the relative telomere length of T-cell products manufactured with IL-7 and IL-15. Note that the same correlations with CD28 expression may not be found in the context of CD4+ T-cells, which may retain CD28 expression at a higher level during aging as compared with CD8+ T cells.

Example3

*CD28 expression on CD8+ T-cells is associated with biased proliferation of T-cell clones*

*Increased CD28 expression in starting PBMCs confers advantageous growth dynamics during T-cell manufacturing*

To characterize the expansion of clonal populations during the T-cell expansion, the expansion kinetics of individual T-cell clones was tracked by clonal DNA sequencing and absolute numbers within each clonal population during the manufacturing process. When tracking individual clones, the clonal divisions as well as the absolute numbers of T cells within a T-cell clonal population were measured during the early (day 4), mid (day 7), and late (day 10) of the expansion process.

For example, to characterize the dynamics of clonal T-cell expansion and contraction via CDR3 DNA sequencing of CD8+ T cells based on CD28$^{low}$ (31.1%), CD28$^{mid}$ (54.3%), or CD28$^{high}$ (93.4%) expression levels, PBMCs were stimulated with agonistic CD3/CD28 antibodies overnight, mock transduced, and then sampled during the expansion process at day 4, day 7, and day 10 in manufacturing process. Since cell counts were performed at each sampling point and the number of T-cells was calculated within each clonal population, the number of clonal divisions may be calculated using the following formula:

$$\text{Clonal Fold Expansion} = (\text{Final Clone \# / Starting Clone \#})$$

$$\text{Estimated Divisions Per Clone} = \text{Log2(Clonal Fold Expansion)}.$$

In addition to quantifying the expansion of certain CD8+ T-cell populations, the contraction of clonal populations may also be quantified, which may not be possible using proliferation dye-based techniques.

FIG. 2 shows CD28$^{high}$ (93.4%) in starting PBMCs conferred an early growth advantage, with nearly two-thirds (63.41%) of T-cell clones expanding between the activation step (day 2) and day 4 in manufacturing. In contrast, lower CD28 expressing starting populations displayed a kinetics, in which most T-cell clones contracted during this early stage of manufacturing, with the CD28$^{mid}$ (54.3%) and the CD28$^{low}$ (31.1%) populations containing 23.74% and 1.19% of early expanding clones, respectively. That is, 76.26% and 98.81% of the CD28$^{mid}$ and CD28$^{low}$ expressing samples contracted, respectively, during the early expansion phase. This was consistent with a negative fold expansion during this phase for these two populations, while the CD28$^{high}$ sample demonstrated a positive fold expansion. Thus, by characterizing T-cell manufacturing at a clonal level, there may be a significant contraction of T-cell clones early in the expansion process, which may be inversely correlated with the starting percentage of CD28-expressing CD8+ T cells.

*Contraction and expansion of clones correlates with starting CD28 percentage*

From the single culture, individual T-cell clonal frequencies were tracked and compared to the post-activation (day 2) time-point. From this comparison, clones which significantly went up and down in frequency were assessed with the sum being the percentage that were differentially abundant.

FIG. 3 shows a strong correlation between the percent differentially abundant and the starting CD28 percentage ($R^2$ = 0.9726). The lower the amount of CD28 in the starting sample, the higher the percentage of clones which became differentially abundant. This suggests that the lack of CD28 in a certain population creates an ecological niche for other clones to grow into and the lack of CD28 creates populations that may die during the T-cell expansion elongated protocol.

*Lower CD28 expressing donors show a delayed T-cell expansion with negative median clonal divisions*

If the CD28 bottleneck exists in the T-cell culture, there would be an expected delay in the T-cell population expansion based on the starting percentage of cells which expressed CD28. Likewise, if all the T-cell clones were able to expand right away, then one would expect a positive number of divisions per clone early in the expansion protocol.

FIG. 4 shows, for the low and medium CD28 expressing cultures, e.g., CD28$^{mid}$ and CD28$^{low}$, there was a negative population growth between the post-activation (day 2) and day 4 into the expansion, this suggests a contraction in the number of cells between these two time points and meets the definition of a bottleneck event. Additionally, only for the high CD28 expressing cultures, e.g., CD28$^{high}$, an overall positive clonal divisions was observed, indicating that in this culture a high percentage of the T-cell clones were able to immediately divide.

FIG. 5 shows, as tracking the divisions of the clonal populations, the CD28$^{low}$ sample displays a non-normally distributed division pattern at the end of the expansion, while the CD28$^{mid}$ and CD28$^{high}$ population show a more normally distributed characterization, i.e., a normal distribution of clonal divisions throughout the manufacturing, as indicated by the similar average and median clonal divisions.

Concordant with increased contraction and reduced early expansion, CD28$^{low}$ populations may require an increased number of clonal divisions to reach a given level of expansion in culture. That is, the lower the starting CD28 expression, the more divisions it may take to reach the same number of T-cells.

FIG. 6 shows the CD28$^{low}$ population required 1.96 clonal divisions to reach expansion of $1 \times 10^8$ cells, while the CD28$^{mid}$ population required 1.64 clonal divisions and the CD28$^{high}$ population divided only 0.96 times for the same number of cells.

Together, this data indicates that high CD28 starting populations may undergo a more advantageous T-cell expansion marked by reduced T-cell contraction and a lower number of necessary T-cell divisions.

These results suggest that the higher the CD28 expression of T-cell populations, the greater the probability of early T-cell clonal expansion. Additionally, the reduced number of T-cell divisions per defined number of cells suggests that an increased expression of CD28 may preserve T-cell proliferation potential.

To determine whether the early expansion of certain clonal populations can be sustained throughout the expansion process, the average final clonal divisions between T-cell clones, e.g., CD28$^{high}$, CD28$^{mid}$, and CD28$^{low}$, that underwent a positive or negative early expansion (day 2 to day 4) was calculated.

FIG. 7 shows, in all T-cell populations irrespective of CD28 expression, early expanding clones were statistically more likely to divide by the end of the expansion process (day 2 to day 4).

*Reduction of unique T-cell clones during expansion in donors with lower expression of CD28 by DNA clonal sequencing*

During the activation phase of manufacturing, activation-induced cell death (AICD) may occur and younger, more naive-like T cells may have higher proliferation potential as compared to older effector-like cells. Thus, these factors may lead to bottlenecks in T-cell manufacturing, e.g., removing T-cell clonal populations from the total population, while others retained in the final product. To investigate the effect of AICD on T cell products, the clonal diversity (or richness) throughout the manufacturing process was determined as a measure for the relative number of unique T-cell clonal populations.

Tracking the number of unique T-cell clones throughout a culture, one would expect that, if the bottleneck exist, there would be large swings in the number of unique T-cell clones post-bottleneck event. Clonal richness may be the measurement for calculating the number of unique T-cell clones normalized to the number of DNA molecule reads from sequencing.

FIG. 8 shows, for all T-cell populations irrespective of CD28 expression, there was an increase in clonal richness (or clonal diversity) from post-activation (day 2) to early expansion (day 4), likely representing the expansion of previously undetectable, low frequency clones. Note that maximal clonal diversity may be achieved at this early stage of the expansion process, a metric may be associated with improved clinical responses to checkpoint therapy and chemotherapy. Following this early burst, a significant decrease in clonal diversity for the CD28$^{low}$ and CD28$^{mid}$-expressing populations, representing the contraction of unique T-cell clones unable to survive the manufacturing process. This reduction in clonal diversity between the early and late expansion time points (day 4 to day 10) suggests that there was considerable clonal elimination as the expansion continued. In contrast, the CD28$^{high}$ population retained a similar level of clonality throughout the manufacturing process. These results suggest that CD28 expression levels in the starting PBMC culture may be strongly associated with divergent expansion kinetics of the CD8+ T-cell compartment. These results may impact T-cell product efficacy because a lack of clonal diversity was associated with poor 4 year survival in diffuse large B-cell lymphoma (DLBCL). T-cell persistence may contribute to T-cell product efficacy. Weak non-cognate TCR-pMHC interactions may contribute to the homeostatic proliferation and persistence of T-cells. Thus, disadvantages of lack of clonal diversity in final T-cell products, such as those prepared from starting T cells with lower CD28 expression, may include reduced T-cell homeostatic proliferation due to a reduced probability of encountering self-sustaining non-cognate TCR-pMHC survival signals.

Example 4

*Younger patients have an improved response to CD19 CAR therapy when manufactured with CD28 co-stimulation*

To further explore the significance of the proposed ex vivo T-cell expansion bottleneck, a clinical trial meta-analysis was performed to investigate whether the loss of CD28 expression in elderly patients would create clinical trends. Based on the previous data, older patients would perform differently compared to younger patients based on the T cell manufacturing (CD3 alone or in conjunction with CD28).

40 T cell therapy clinical trials were published. Many of them are early stage trials with unconfirmed moieties (e.g. an untested new TCR or CAR molecule). The meta-analysis required filtering steps to create a uniform comparable data set. After filtering and compilation, 7 clinical trials targeting CD19 malignancies for a total for 107 patient data points were analyzed. From this analysis, patients younger than 45 had a better clinical prognosis (66.67%) when their cells were manufactured with CD3 + CD28 method as compared with CD3 alone (44.44%). In contrast, when patients were older than 45, there was a benefit to being manufactured with CD3 alone (71.43%) rather than the CD3 + CD28 method (30.00%) (Table 1).

Table 1

|  |  | CR | PR | SD | NR/PD | NE | Total | ORR (PR + CR ) | Clinical Trials |
|---|---|---|---|---|---|---|---|---|---|
| Patients >45 Years of Age |  |  |  |  |  |  |  |  |  |
|  | + CD28 | 2 | 1 | 4 | 1 | 2 | 10 | 30.00% | Lee2014A,Brentjens2011 |
|  | - CD28 | 3 | 2 |  | 2 |  | 7 | 71.43% | Dai2015,Locke2017 |
|  |  |  |  |  |  |  |  |  |  |
| Patients <45 Years of Age |  |  |  |  |  |  |  |  |  |
|  | +CD28 | 14 |  | 3 | 4 |  | 21 | 66.67% | Lee2014A,Brentjens2011 |
|  | -CD28 | 3 | 1 |  | 2 | 3 | 9 | 44.44% | Dai2015,Locke2017 |
|  |  |  |  |  | Total |  | 47 |  |  |

Table 1: The differential effects of CD28 costimulation. Data is from the meta-analysis of 4 CD19 CAR trials with 47 total data points. Patients younger than 45 performed better when given CD28 costimulation in the manufacturing as compared to patients older than 45, who performed better when not given CD28 costimulation. In other words, patients younger than 45 may benefit from a CD3 + CD28 method of T-cell manufacturing, while patients older than 45 may benefit from a CD3 only method of T-cell manufacturing. CR = complete response, PR = partial response, SD = stable disease, NR/PD = no response/progressive disease, NE = not evaluable. Each clinical response was defined based on the source clinical trial analysis.

This meta-analysis of available clinical trial data shows that younger patients, e.g., younger than 45, appear to respond better to T-cell manufacturing involving CD28 costimulation, while older patients, e.g., older than 45, appear to respond better to T-cell manufacturing lacking CD28 costimulation.

*Clinical response rates correlate with the fold growth ex vivo in clinical trial against multiple myeloma*

Multiple clinical and preclinical investigations suggest that phenotypically younger less differentiated T cells outperform in comparison to older more differentiated T cell products. Based on the manufacturing data (FIGS. 1 and 2), high CD28 expressing (younger) starting PBMCs may achieve a higher fold expansion ex vivo and yield a phenotypically less differentiated final product. Thus, a less differentiated, and a more potent clinical product may be obtained by manufacturing T cells using more youthful, less differentiated starting PBMCs for the same period and culturing them to achieve a higher fold expansion.

Table 2 shows, from a αBCMA multiple myeloma CAR clinical trial, there was a 57% response rate when cell cultures achieved greater than 10-fold expansion ex vivo. In comparison, there was a 0% response rate when cultures failed to achieve 10-fold expansion. These observations further support the translational relevance and manufacturing centric model in predicting T cell potency.

Table 2

| Disease | Target | Age | Dosage (10^9) | Response | Fold Expansion Day 9 | Response Rate |
|---------|--------|-----|---------------|----------|---------------------|---------------|
| MM | BCMA | NA | 0.0003/kg | PR | 28.96 | 57% Response Rate |
| MM | BCMA | NA | 0.001/kg | SD | 24.89 | |
| MM | BCMA | NA | 0.009/kg | CR | 15.00 | |
| MM | BCMA | NA | 0.009/kg | SD | 13.92 | |
| MM | BCMA | NA | 0.003/kg | PR | 13.63 | |
| MM | BCMA | NA | 0.0003/kg | SD | 11.99 | |
| MM | BCMA | NA | 0.009/kg | PR | 10.96 | |
| MM | BCMA | NA | 0.0003/kg | SD | 9.91 | 0% Response Rate |
| MM | BCMA | NA | 0.003/kg | SD | 9.35 | |

| Disease | Target | Age | Dosage (10^9) | Response | Fold Expansion Day 9 | Response Rate |
|---------|--------|-----|---------------|----------|---------------------|---------------|
| MM | BCMA | NA | 0.001/kg | SD | 5.62 | |
| MM | BCMA | NA | 0.003/kg | SD | 3.85 | |
| MM | BCMA | NA | 0.001/kg | SD | 2.72 | |

Table 2: Ex vivo manufacturing metrics correlate with clinical response in multiple myeloma. Data from clinical manufacturing were combined with the clinical response rates and sorted by the fold expansion of CD3+ cells achieved during manufacturing. Response rates were calculated as the number of patients who achieved a PR or CR in relation to the total number of patients in the group. PR = partial response, SD = stable disease, CR = complete response.

Advantages of the present disclosure may include prediction of the final CD8%, the fold expansion, and the relative telomere length of T-cell products by measuring the CD28 expression of the starting CD8+ T-cells, personalized therapy based on CD28 expression in starting % of CD28+ CD8+ T cell populations. In addition, the manufacturing of the present disclosure may be personalized with variable manufacturing periods, starting cell numbers, stimulation conditions, and different growth mediums. This may improve in vitro manufacturing metrics, e.g., fold expansion, and may be correlated with better clinical outcome. Cell therapy manufacturing of the present disclosure may be highly patient specific with specific groups responding better or worse to manufacturing based on their starting cellular phenotype.

**Claims**

1. A method for producing T cells with improved efficacy for adoptive immunotherapy comprising

   obtaining a population of CD8+ T cells from a patient or a donor,
   determining a % of CD28+ CD8+ T cells in the obtained population, and
   activating the determined population with anti-CD3 antibody and anti-CD28 antibody, provided that the determined population comprises at least about 50% of CD28+ CD8+ T cells, preferably at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% of CD28+ CD8+ T cells, or
   activating the determined population with anti-CD3 antibody in the absence of anti-CD28 antibody, provided that the determined population comprises less than about 50% of CD28+ CD8+ T cells, preferably less than about 40%, less than about 35%, less than about 30%, less than about 25%, less than about 20%, less than about 15%, less than about 10%, less than about 9%, less than about 8%, less than about 7%, less than about 6%, less than about 5%, less than about 4%, less than about 3%, less than about 2%, or less than about 1% of CD28+ CD8+ T cells,
   transducing the activated T cell population with a viral vector, preferably a retroviral or lentiviral vector expressing a T cell receptor (TCR) or a chimeric antigen receptor (CAR), and
   expanding the transduced T cell population.

2. A method for producing T cells with improved efficacy for adoptive immunotherapy comprising

   obtaining a population of CD8+ T cells from a patient or a donor, and
   isolating CD28+ CD8+ T cells from the obtained population,
   wherein the isolated cells comprise at least 50% of CD28+ CD8+ T cells, preferably wherein the isolated cells comprise at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% of CD28+ CD8+ T cells,
   activating the isolated cells with anti-CD3 antibody and anti-CD28 antibody, transducing the activated T cell population with a viral vector, preferably a retroviral or lentiviral vector expressing a T cell receptor (TCR) or a chimeric antigen receptor (CAR), and
   expanding the transduced T cell population.

3. The method of claim 1 or 2, wherein the transducing and the expanding are carried out in the presence of at least one cytokine, preferably wherein the at least one cytokine is selected from interleukin (IL)-2, IL-7, IL-10, IL-12, IL-15, and IL-21, more preferably wherein the at least one cytokine comprises IL-7 and IL-15, most preferably wherein the concentration of IL-7 is from about 1 ng/ml to 90 ng/ml, about 1 ng/ml to 80 ng/ml, about 1 ng/ml to 70 ng/ml, about 1 ng/ml to 60 ng/ml, about 1 ng/ml to 50 ng/ml, about 1 ng/ml to 40 ng/ml, about 1 ng/ml to 30 ng/ml, about 1 ng/ml to 20 ng/ml, about 1 ng/ml to 15 ng/ml, about 1 ng/ml to 10 ng/ml, about 2 ng/ml to 10 ng/ml, about 4 ng/ml to 10 ng/ml, about 6 ng/ml to 10 ng/ml, or about 5 ng/ml to 10 ng/ml and/or the concentration of IL-15 is from about 5 ng/ml to 500 ng/ml, about 5 ng/ml to 400 ng/ml, about 5 ng/ml to 300 ng/ml, about 5 ng/ml to 200 ng/ml, about 5 ng/ml to 150 ng/ml, about 5 ng/ml to 100 ng/ml, about 10 ng/ml to 100 ng/ml, about 20 ng/ml to 100 ng/ml, about 30 ng/ml to 100 ng/ml, about 40 ng/ml to 100 ng/ml, about 50 ng/ml to 100 ng/ml, about 60 ng/ml to 100 ng/ml, about 70 ng/ml to 100 ng/ml, about 80 ng/ml to 100 ng/ml, about 90 ng/ml to 100 ng/ml, about 10 ng/ml to 50 ng/ml, about 20 ng/ml to 50 ng/ml, about 30 ng/ml to 50 ng/ml, or about 40 ng/ml to 50 ng/ml.

4. The method of any one of claims 1-3, wherein

   - the transducing is carried out within a period of from about 1 hour to 120 hours, about 1 hour to 108 hours, about 1 hour to 96 hours, about 1 hour to 72 hours, about 1 hour to 48 hours, about 1 hour to 36 hours, about 1 hour to 24 hours, about 2 hours to 24 hours, about 4 hours to 24 hours, about 6 hours to 24 hours, about 8 hours to 24 hours, about 10 hours to 24 hours, about 12 hours to 24 hours, about 14 hours to 24 hours, about 16 hours to 24 hours, about 18 hours to 24 hours, about 20 hours to 24 hours, or about 22 hours to 24 hours;
   - the expanding is carried out within a period of from about 1 day to about 30 days, about 1 day to about 25 days, about 1 day to about 20 days, about 1 day to about 15 days, about 1 day to about 10 days, about 2 days to about 10 days, about 3 days to about 10 days, about 4 days to about 10 days, about 5 days to about 10 days, about 6 days to about 10 days, about 7 days to about 10 days, about 8 days to about 10 days, or about 9 days to about 10 days,

- the activation is carried out within a period of from about 1 hour to about 120 hours, about 1 hour to about 108 hours, about 1 hour to about 96 hours, about 1 hour to about 84 hours, about 1 hour to about 72 hours, about 1 hour to about 60 hours, about 1 hour to about 48 hours, about 1 hour to about 36 hours, about 1 hour to about 24 hours, about 2 hours to about 24 hours, about 4 hours to about 24 hours, about 6 hours to about 24 hours, about 8 hours to about 24 hours, about 10 hours to about 24 hours, about 12 hours to about 24 hours, about 12 hours to about 72 hours, about 24 hours to about 72 hours, about 6 hours to about 48 hours, about 24 hours to about 48 hours, about 6 hours to about 72 hours, or about 1 hour to about 12 hours.

5. The method of any one of claims 1-4, wherein the activating comprises immobilizing the T cells with the anti-CD3 antibody and/or the anti-CD28 antibody on a solid phase support, preferably wherein the anti-CD3 antibody and the anti-CD28 antibody each have a concentration of from about 0.1 $\mu$g/ml to about 10.0 $\mu$g/ml, about 0.1 $\mu$g/ml to about 8.0 $\mu$g/ml, about 0.1 $\mu$g/ml to about 6.0 $\mu$g/ml, about 0.1 $\mu$g/ml to about 4.0 $\mu$g/ml, about 0.1 $\mu$g/ml to about 2.0 $\mu$g/ml, about 0.1 $\mu$g/ml to about 1.0 $\mu$g/ml, about 0.1 $\mu$g/ml to about 0.8 $\mu$g/ml, about 0.1 $\mu$g/ml to about 0.6 $\mu$g/ml, about 0.1 $\mu$g/ml to about 0.5 $\mu$g/ml, about 0.1 $\mu$g/ml to about 0.25 $\mu$g/ml, about 0.2 $\mu$g/ml to about 0.5 $\mu$g/ml, about 0.2 $\mu$g/ml to about 0.3 $\mu$g/ml, about 0.3 $\mu$g/ml to about 0.5 $\mu$g/ml, about 0.3 $\mu$g/ml to about 0.4 $\mu$g/ml, or about 0.4 $\mu$g/ml to about 0.5 $\mu$g/ml.

6. A genetically transduced T cell population for use in a method of treating cancer, wherein the method of treating cancer comprises the steps of

determining a % of CD28+ CD8+ T cells in a population of CD8+ T cells obtained from a patient, activating the determined population with anti-CD3 antibody and anti-CD28 antibody, provided that the determined population comprises at least about 50% of CD28+ CD8+ T cells, or activating the determined population with anti-CD3 antibody in the absence of anti-CD28 antibody, provided that the determined population comprises less than about 50% of CD28+ CD8+ T cells, transducing the activated T cell population with a viral vector, expanding the transduced T cell population, and administering to the patient the expanded T cell population,

and wherein the cancer is selected from the group consisting of hepatocellular carcinoma (HCC), colorectal carcinoma (CRC), glioblastoma (GB), gastric cancer (GC), esophageal cancer, non-small cell lung cancer (NSCLC), pancreatic cancer (PC), renal cell carcinoma (RCC), benign prostate hyperplasia (BPH), prostate cancer (PCA), ovarian cancer (OC), melanoma, breast cancer, chronic lymphocytic leukemia (CLL), Merkel cell carcinoma (MCC), small cell lung cancer (SCLC), Non-Hodgkin lymphoma (NHL), acute myeloid leukemia (AML), gallbladder cancer and cholangiocarcinoma (GBC, CCC), urinary bladder cancer (UBC), acute lymphoblastic leukemia (ALL), multiple myeloma (MM), and uterine cancer (UEC).

7. The genetically transduced T cell population for use of claim 6, wherein the method of treating cancer comprises

determining a fold expansion of the expanded T cell population, administering to the patient the expanded T cell population, provided that the fold expansion is greater than 10-fold.

**Patentansprüche**

1. Verfahren zum Herstellen von T-Zellen mit verbesserter Wirksamkeit für die adoptive Immuntherapie, umfassend:

Erhalten einer Population von CD8+-T-Zellen von einem Patienten oder einem Spender, Bestimmen eines Prozentsatzes von CD28+-CD8+-T-Zellen in der erhaltenen Population und Aktivieren der bestimmten Population mit einem Anti-CD3-Antikörper und einem Anti-CD28-Antikörper, vorausgesetzt, dass die bestimmte Population mindestens etwa 50 % CD28+-CD8+-T-Zellen umfasst, vorzugsweise mindestens etwa 55 %, mindestens etwa 60 %, mindestens etwa 65 %, mindestens etwa 70 %, mindestens etwa 75 %, mindestens etwa 80 %, mindestens etwa 85 %, mindestens etwa 90 %, mindestens etwa 91 %, mindestens etwa 92 %, mindestens etwa 93 %, mindestens etwa 94 %, mindestens etwa 95 %, mindestens etwa 96 %, mindestens etwa 97 %, mindestens etwa 98 % oder mindestens etwa 99 % CD28+-CD8+-T-Zellen, oder

Aktivieren der bestimmten Population mit einem Anti-CD3-Antikörper in Abwesenheit von einem Anti-CD28-Antikörper, vorausgesetzt, dass die bestimmte Population weniger als etwa 50 % CD28+-CD8+-T-Zellen umfasst, vorzugsweise weniger als etwa 40 %, weniger als etwa 35 %, weniger als etwa 30 %, weniger als etwa 25 %, weniger als etwa 20 %, weniger als etwa 15 %, weniger als etwa 10 %, weniger als etwa 9 %, weniger als etwa 8 %, weniger als etwa 7 %, weniger als etwa 6 %, weniger als etwa 5 %, weniger als etwa 4 %, weniger als etwa 3 %, weniger als etwa 2 % oder weniger als etwa 1 % CD28+-CD8+-T-Zellen, Transduzieren der aktivierten T-Zell-Population mit einem viralen Vektor, vorzugsweise einem retroviralen oder lentiviralen Vektor, der einen T-Zell-Rezeptor (TCR) oder einen chimären Antigenrezeptor (CAR) exprimiert, und Expandieren der transduzierten T-Zell-Population.

2. Verfahren zum Herstellen von T-Zellen mit verbesserter Wirksamkeit für die adoptive Immuntherapie, umfassend:

Erhalten einer Population von CD8+-T-Zellen von einem Patienten oder Spender und Isolieren von CD28+-CD8+-T-Zellen aus der erhaltenen Population, wobei die isolierten Zellen mindestens 50 % CD28+-CD8+-T-Zellen umfassen, wobei die isolierten Zellen vorzugsweise Folgendes umfassen: mindestens etwa 55 %, mindestens etwa 60 %, mindestens etwa 65 %, mindestens etwa 70 %, mindestens etwa 75 %, mindestens etwa 80 %, mindestens etwa 85 %, mindestens etwa 90 %, mindestens etwa 91 %, mindestens etwa 92 %, mindestens etwa 93 %, mindestens etwa 94 %, mindestens etwa 95 %, mindestens etwa 96 %, mindestens etwa 97 %, mindestens etwa 98 % oder mindestens etwa 99 % CD28+-CD8+-T-Zellen, Aktivieren der isolierten Zellen mit einem Anti-CD3-Antikörper und einem Anti-CD28-Antikörper, Transduzieren der aktivierten T-Zell-Population mit einem viralen Vektor, vorzugsweise einem retroviralen oder lentiviralen Vektor, der einen T-Zell-Rezeptor (TCR) oder einen chimären Antigenrezeptor (CAR) exprimiert, und Expandieren der transduzierten T-Zell-Population.

3. Verfahren nach Anspruch 1 oder 2, wobei das Transduzieren und Expandieren in Gegenwart mindestens eines Zytokins durchgeführt werden, wobei das mindestens eine Zytokin vorzugsweise aus Interleukin (IL)-2, IL-7, IL-10, IL-12, IL-15 und IL-21 ausgewählt ist, wobei das mindestens eine Zytokin besonders bevorzugt IL-7 und IL-15 umfasst, wobei die Konzentration von IL-7 am meisten bevorzugt Folgendes beträgt: etwa 1 ng/ml bis 90 ng/ml, etwa 1 ng/ml bis 80 ng/ml, etwa 1 ng/ml bis 70 ng/ml, etwa 1 ng/ml bis 60 ng/ml, etwa 1 ng/ml bis 50 ng/ml, etwa 1 ng /ml bis 40 ng/ml, etwa 1 ng/ml bis 30 ng/ml, etwa 1 ng/ml bis 20 ng/ml, etwa 1 ng/ml bis 15 ng/ml, etwa 1 ng/ml bis 10 ng/ml, etwa 2 ng/ml bis 10 ng/ml, etwa 4 ng/ml bis 10 ng/ml, etwa 6 ng/ml bis 10 ng/ml oder etwa 5 ng/ml bis 10 ng/ml und/oder die Konzentration von IL-15 Folgendes beträgt: etwa 5 ng/ml bis 500 ng/ml, etwa 5 ng/ml bis 400 ng/ml, etwa 5 ng/ml bis 300 ng/ml, etwa 5 ng/ml bis 200 ng/ml. ml, etwa 5 ng/ml bis 150 ng/ml, etwa 5 ng/ml bis 100 ng/ml, etwa 10 ng/ml bis 100 ng/ml, etwa 20 ng/ml bis 100 ng/ml, etwa 30 ng/ml bis 100 ng/ml, etwa 40 ng/ml bis 100 ng/ml, etwa 50 ng/ml bis 100 ng/ml, etwa 60 ng/ml bis 100 ng/ml, etwa 70 ng/ml bis 100 ng/ml, etwa 80 ng/ml bis 100 ng/ml, etwa 90 ng/ml bis 100 ng/ml, etwa 10 ng/ml bis 50 ng/ml, etwa 20 ng/ml bis 50 ng/ml, etwa 30 ng/ml bis 50 ng/ml oder etwa 40 ng/ml bis 50 ng/ml.

4. Verfahren nach einem der Ansprüche 1-3, wobei:

- das Transduzieren innerhalb eines folgenden Zeitraums ausgeführt wird: von etwa 1 Stunde bis 120 Stunden, etwa 1 Stunde bis 108 Stunden, etwa 1 Stunde bis 96 Stunden, etwa 1 Stunde bis 72 Stunden, etwa 1 Stunde bis 48 Stunden, etwa 1 Stunde bis 36 Stunden, etwa 1 Stunde bis 24 Stunden, etwa 2 Stunden bis 24 Stunden, etwa 4 Stunden bis 24 Stunden, etwa 6 Stunden bis 24 Stunden, etwa 8 Stunden bis 24 Stunden, etwa 10 Stunden bis 24 Stunden, etwa 12 Stunden bis 24 Stunden, etwa 14 Stunden bis 24 Stunden, etwa 16 Stunden bis 24 Stunden, etwa 18 Stunden bis 24 Stunden, etwa 20 Stunden bis 24 Stunden oder etwa 22 Stunden bis 24 Stunden;
- das Expandieren innerhalb eines folgenden Zeitraums ausgeführt wird: von etwa 1 Tag bis etwa 30 Tagen, etwa 1 Tag bis etwa 25 Tagen, etwa 1 Tag bis etwa 20 Tagen, etwa 1 Tag bis etwa 15 Tagen, etwa 1 Tag bis etwa 10 Tagen, etwa 2 Tagen bis etwa 10 Tagen, etwa 3 Tagen bis etwa 10 Tagen, etwa 4 Tagen bis etwa 10 Tagen, etwa 5 Tagen bis etwa 10 Tagen, etwa 6 Tagen bis etwa 10 Tagen, etwa 7 Tagen bis etwa 10 Tagen, etwa 8 Tagen bis etwa 10 Tagen oder etwa 9 Tagen bis etwa 10 Tagen,
- das Aktivieren innerhalb eines folgenden Zeitraums ausgeführt wird: von etwa 1 Stunde bis etwa 120 Stunden, etwa 1 Stunde bis etwa 108 Stunden, etwa 1 Stunde bis etwa 96 Stunden, etwa 1 Stunde bis etwa 84 Stunden, etwa 1 Stunde bis etwa 72 Stunden, etwa 1 Stunde bis etwa 60 Stunden, etwa 1 Stunde bis etwa 48 Stunden, etwa 1 Stunde bis etwa 36 Stunden, etwa 1 Stunde bis etwa 24 Stunden, etwa 2 Stunden bis etwa 24 Stunden, etwa 4 Stunden bis etwa 24 Stunden, etwa 6 Stunden bis etwa 24 Stunden, etwa 8 Stunden bis etwa 24 Stunden, etwa 10 Stunden bis etwa 24 Stunden, etwa 12 Stunden bis etwa 24 Stunden, etwa 12 Stunden bis etwa 72 Stunden, etwa 24 Stunden bis etwa 72 Stunden, etwa 6 Stunden bis etwa 48 Stunden, etwa 24 Stunden bis etwa 48 Stunden, etwa 6 Stunden bis etwa 72 Stunden oder etwa 1 Stunde bis etwa 12 Stunden.

**5.** Verfahren nach einem der Ansprüche 1-4, wobei das Aktivieren ein Immobilisieren der T-Zellen mit dem Anti-CD3-Antikörper und/oder dem Anti-CD28-Antikörper auf einem Festphasenträger umfasst, wobei der Anti-CD3-Antikörper und der Anti-CD28-Antikörper vorzugsweise jeweils eine folgende Konzentration aufweisen: von etwa 0,1 $\mu$g/ml bis etwa 10,0 $\mu$g/ml, etwa 0,1 $\mu$g/ml bis etwa 8,0 $\mu$g/ml, etwa 0,1 $\mu$g/ml bis etwa 6,0 $\mu$g/ml, etwa 0,1 $\mu$g/ml bis etwa 4,0 $\mu$g/ml, etwa 0,1 $\mu$g/ml bis etwa 2,0 $\mu$g/ml, etwa 0,1 $\mu$g/ml bis etwa 1,0 $\mu$g/ml, etwa 0,1 $\mu$g/ml bis etwa 0,8 $\mu$g/ml, etwa 0,1 $\mu$g/ml bis etwa 0,6 $\mu$g/ml, etwa 0,1 $\mu$g/ml bis etwa 0,5 $\mu$g/ml, etwa 0,1 $\mu$g/ml bis etwa 0,25 $\mu$g/ml, etwa 0,2 $\mu$g/ml bis etwa 0,5 $\mu$g/ml, etwa 0,2 $\mu$g/ml bis etwa 0,3 $\mu$g/ml, etwa 0,3 $\mu$g/ml bis etwa 0,5 $\mu$g/ml, etwa 0,3 $\mu$g/ml bis etwa 0,4 $\mu$g/ml oder etwa 0,4 $\mu$g/ml bis etwa 0,5 $\mu$g/ml.

**6.** Genetisch transduzierte T-Zell-Population zur Verwendung in einem Verfahren zum Behandeln von Krebs, wobei das Verfahren zum Behandeln von Krebs die folgenden Schritte umfasst:

Bestimmen eines Prozentsatzes von CD28+-CD8+-T-Zellen in einer Population von CD8+-T-Zellen, die von einem Patienten erhalten wurden,
Aktivieren der bestimmten Population mit einem Anti-CD3-Antikörper und einem Anti-CD28-Antikörper, vorausgesetzt, dass die bestimmte Population mindestens etwa 50 % CD28+-CD8+-T-Zellen umfasst, oder
Aktivieren der bestimmten Population mit einem Anti-CD3-Antikörper in Abwesenheit von einem Anti-CD28-Antikörper, vorausgesetzt, dass die bestimmte Population weniger als etwa 50 % CD28+-CD8+-T-Zellen umfasst,
Transduzieren der aktivierten T-Zell-Population mit einem viralen Vektor,
Expandieren der transduzierten T-Zell-Population und
Verabreichen der expandierten T-Zell-Population an den Patienten
und wobei der Krebs aus der Gruppe ausgewählt ist, die aus Folgendem besteht: hepatozellulärem Karzinom (HCC), kolorektalem Karzinom (CRC), Glioblastom (GB), Magenkrebs (GC), Speiseröhrenkrebs, nicht-kleinzelligem Lungenkrebs (NSCLC), Bauchspeicheldrüsenkrebs (PC), Nierenzellkarzinom (RCC), benigner Prostatahyperplasie (BPH), Prostatakrebs (PCA), Eierstockkrebs (OC), Melanom, Brustkrebs, chronischer lymphatischer Leukämie (CLL), Merkelzellkarzinom (MCC), kleinzelligem Lungenkrebs (SCLC), Non-Hodgkin-Lymphom (NHL), akuter myeloischer Leukämie (AML), Gallenblasenkrebs und Cholangiokarzinom (GBC, CCC), Harnblasenkrebs (UBC), akuter lymphoblastischer Leukämie (ALL), Multiplem Myelom (MM) und Gebärmutterkrebs (UEC).

**7.** Genetisch transduzierte T-Zell-Population zur Verwendung nach Anspruch 6, wobei das Verfahren zum Behandeln von Krebs Folgendes umfasst:

Bestimmen einer Expansion der expandierten T-Zell-Population um ein Vielfaches,
Verabreichen der expandierten T-Zell-Population an den Patienten, vorausgesetzt, dass die Expansion um ein Vielfaches mehr als das 10-fache beträgt.

**Revendications**

**1.** Procédé de production de lymphocytes T avec une efficacité améliorée pour l'immunothérapie adoptive comprenant

l'obtention d'une population de lymphocytes T CD8+ à partir d'un patient ou d'un donneur,
la détermination d'un % de lymphocytes T CD28+ CD8+ dans la population obtenue, et
l'activation de la population déterminée avec un anticorps anti-CD3 et un anticorps anti-CD28, à condition que la population déterminée comprenne au moins environ 50 % de lymphocytes T CD28+ CD8+, de préférence au moins environ 55 %, au moins environ 60 %, au moins environ 65 %, au moins environ 70 %, au moins environ 75 %, au moins environ 80 %, au moins environ 85 %, au moins environ 90 %, au moins environ 91 %, au moins environ 92 %, au moins environ 93 %, au moins environ 94 %, au moins environ 95 %, au moins environ 96 %, au moins environ 97 %, au moins environ 98 %, ou au moins environ 99 % de lymphocytes T CD28+ CD8+, ou
l'activation de la population déterminée avec un anticorps anti-CD3 en l'absence d'anticorps anti-CD28, à condition que la population déterminée comprenne moins d'environ 50 % de lymphocytes T CD28+ CD8+, de préférence moins d'environ 40 %, moins d'environ 35 %, moins de environ 30 %, moins d'environ 25 %, moins d'environ 20 %, moins d'environ 15 %, moins d'environ 10 %, moins d'environ 9 %, moins d'environ 8 %, moins d'environ 7 %, moins d'environ 6 %, moins d'environ 5 %, moins d'environ 4 %, moins d'environ 3 %, moins d'environ 2 %, ou moins d'environ 1 % de lymphocytes T CD28+ CD8+,
la transduction de la population de lymphocytes T activés avec un vecteur viral, de préférence un vecteur rétroviral ou lentiviral exprimant un récepteur de lymphocyte T (TCR) ou un récepteur d'antigène chimérique (CAR), et
l'expansion de la population de lymphocytes T transduits.

2. Procédé de production de lymphocytes T avec une efficacité améliorée pour l'immunothérapie adoptive comprenant

l'obtention d'une population de lymphocytes T CD8+ à partir d'un patient ou d'un donneur, et
l'isolement des lymphocytes T CD28+ CD8+ de la population obtenue,
dans lequel les cellules isolées comprennent au moins 50 % de lymphocytes T CD28+ CD8+, de préférence dans lequel les cellules isolées comprennent au moins environ 55 %, au moins environ 60 %, au moins environ 65 %, au moins environ 70 %, au moins environ 75 % , au moins environ 80 %, au moins environ 85 %, au moins environ 90 %, au moins environ 91 %, au moins environ 92 %, au moins environ 93 %, au moins environ 94 %, au moins environ 95 %, au moins environ 96 %, au moins environ 97 %, au moins environ 98 %, ou au moins environ 99 % de lymphocytes T CD28+ CD8+,
l'activation des cellules isolées avec un anticorps anti-CD3 et un anticorps anti-CD28,
la transduction de la population de cellules T activées avec un vecteur viral, de préférence un vecteur rétroviral ou lentiviral exprimant un récepteur de cellule T (TCR) ou un récepteur d'antigène chimérique (CAR), et
l'expansion de la population de lymphocytes T transduits.

3. Procédé selon la revendication 1 ou 2, dans lequel la transduction et l'expansion sont réalisées en présence d'au moins une cytokine, de préférence dans lequel l'au moins une cytokine est choisie parmi l'interleukine (IL)-2, IL-7, IL-10, IL-12, IL-15 et IL-21, plus préférablement dans lequel l'au moins une cytokine comprend IL-7 et IL-15, plus préférablement dans lequel la concentration d'IL-7 est d'environ 1 ng/ml à 90 ng/ml, environ 1 ng/ml à 80 ng/ml, environ 1 ng/ml à 70 ng/ml, environ 1 ng/ml à 60 ng/ml, environ 1 ng/ml à 50 ng/ml, environ 1 ng/ml à 40 ng/ml, environ 1 ng/ml à 30 ng/ml, environ 1 ng/ml à 20 ng/ml, environ 1 ng/ml à 15 ng/ml, environ 1 ng/ml à 10 ng/ml, environ 2 ng/ml à 10 ng/ml, environ 4 ng/ml à 10 ng/ml, environ 6 ng/ml à 10 ng/ml, ou environ 5 ng/ml à 10 ng/ml et/ou la concentration d'IL-15 est d'environ 5 ng/ml à 500 ng/ml, environ 5 ng/ml à 400 ng/ml, environ 5 ng/ml à 300 ng/ml, environ 5 ng/ml à 200 ng/ml, environ 5 ng/ml à 150 ng/ml, environ 5 ng/ml à 100 ng/ml, environ 10 ng/ml à 100 ng/ml, environ 20 ng/ml à 100 ng/ml, environ 30 ng/ml à 100 ng/ml, environ 40 ng/ml à 100 ng/ml, environ 50 ng/ml à 100 ng/ml, environ 60 ng/ml à 100 ng/ml, environ 70 ng/ml à 100 ng/ml , environ 80 ng/ml à 100 ng/ml, environ 90 ng/ml à 100 ng/ml, environ 10 ng/ml à 50 ng/ml, environ 20 ng/ml à 50 ng/ml, environ 30 ng/ml à 50 ng/ml, ou environ 40 ng/ml à 50 ng/ml.

4. Procédé selon l'une quelconque des revendications 1 à 3, dans lequel

- la transduction est réalisée dans un délai allant d'environ 1 heure à 120 heures, environ 1 heure à 108 heures, environ 1 heure à 96 heures, environ 1 heure à 72 heures, environ 1 heure à 48 heures, environ 1 heure à 36 heures, environ 1 heure à 24 heures, environ 2 heures à 24 heures, environ 4 heures à 24 heures, environ 6 heures à 24 heures, environ 8 heures à 24 heures, environ 10 heures à 24 heures, environ 12 heures à 24 heures, environ 14 heures à 24 heures, environ 16 heures à 24 heures, environ 18 heures à 24 heures, environ 20 heures à 24 heures, ou environ 22 heures à 24 heures ;
- l'expansion est réalisée dans un délai d'environ 1 jour à environ 30 jours, environ 1 jour à environ 25 jours, environ 1 jour à environ 20 jours, environ 1 jour à environ 15 jours, environ 1 jour à environ 10 jours, environ 2 jours à environ 10 jours, environ 3 jours à environ 10 jours, environ 4 jours à environ 10 jours, environ 5 jours à environ 10 jours, environ 6 jours à environ 10 jours, environ 7 jours à environ 10 jours, environ 8 jours à environ 10 jours, ou environ 9 jours à environ 10 jours,
- l'activation est effectuée dans un délai d'environ 1 heure à environ 120 heures, environ 1 heure à environ 108 heures, environ 1 heure à environ 96 heures, environ 1 heure à environ 84 heures, environ 1 heure à environ 72 heures, environ 1 heure à environ 60 heures, environ 1 heure à environ 48 heures, environ 1 heure à environ 36 heures, environ 1 heure à environ 24 heures, environ 2 heures à environ 24 heures, environ 4 heures à environ 24 heures, environ 6 heures à environ 24 heures, environ 8 heures à environ 24 heures, environ 10 heures à environ 24 heures, environ 12 heures à environ 24 heures, environ 12 heures à environ 72 heures, environ 24 heures à environ 72 heures, environ 6 heures à environ 48 heures, environ 24 heures à environ 48 heures, environ 6 heures à environ 72 heures, ou environ 1 heure à environ 12 heures.

5. Procédé selon l'une quelconque des revendications 1 à 4, dans lequel l'activation comprend l'immobilisation des lymphocytes T avec l'anticorps anti-CD3 et/ou l'anticorps anti-CD28 sur un support en phase solide, de préférence dans lequel l'anticorps anti-CD3 et l'anticorps anti-CD28 comportent chacun une concentration d'environ 0,1 µg/ml à environ 10,0 µg/ml, environ 0,1 µg/ml à environ 8,0 µg/ml, environ 0,1 µg/ml à environ 6,0 µg/ml, environ 0,1 µg/ml à environ 4,0 µg/ml, environ 0,1 µg/ml à environ 2,0 µg/ml, environ 0,1 µg/ml à environ 1,0 µg/ml, environ 0,1 µg/ml à environ 0,8 µg/ml, environ 0,1 µg/ml à environ 0,6 µg/ml, environ 0,1 µg/ml à environ 0,5 µg/ml, environ 0,1 µg/ml à environ 0,25 µg/ml, environ 0,2 µg/ml à environ 0,5 µg/ml, environ 0,2 µg/ml à environ 0,3 µg/ ml, environ 0,3 µg/ml à environ 0,5 µg/ml, environ 0,3 µg/ml à environ 0,4 µg/ml, ou environ 0,4 µg/ml à environ 0,5 µg/ml.

6. Population de lymphocytes T génétiquement transduits destinée à être utilisée dans un procédé de traitement du cancer, dans laquelle le procédé de traitement du cancer comprend les étapes de

détermination d'un % de lymphocytes T CD28+ CD8+ dans une population de lymphocytes T CD8+ obtenue à partir d'un patient,
activation de la population déterminée avec un anticorps anti-CD3 et un anticorps anti-CD28, à condition que la population déterminée comprenne au moins environ 50 % de lymphocytes T CD28+ CD8+, ou
activation de la population déterminée avec un anticorps anti-CD3 en l'absence d'anticorps anti-CD28, à condition que la population déterminée comprenne moins d'environ 50 % de lymphocytes T CD28+ CD8+,
transduction de la population de lymphocytes T activés avec un vecteur viral,
expansion de la population de lymphocytes T transduits, et
administration au patient de la population de lymphocytes T expansés,
et dans lequel le cancer est choisi dans le groupe constitué de carcinome hépatocellulaire (HCC), carcinome colorectal (CRC), glioblastome (GB), cancer gastrique (GC), cancer de l'oesophage, cancer du poumon non à petites cellules (NSCLC), cancer du pancréas (PC), carcinome à cellules rénales (RCC), hyperplasie bénigne de la prostate (HBP), cancer de la prostate (PCA), cancer de l'ovaire (CO), mélanome, cancer du sein, leucémie lymphoïde chronique (LLC), carcinome à cellules de Merkel (MCC), cancer du poumon à petites cellules (SCLC), lymphome non hodgkinien (LNH), leucémie myéloïde aiguë (LAM), cancer de la vésicule biliaire et cholangiocarcinome (GBC, CCC), cancer de la vessie (UBC), leucémie aiguë lymphoblastique (ALL), myélome multiple (MM) et cancer de l'utérus (UEC).

7. Population de lymphocytes T génétiquement transduits destinée à être utilisée selon la revendication 6, dans laquelle le procédé de traitement du cancer comprend

la détermination d'un facteur d'expansion de la population de lymphocytes T expansés,
l'administration au patient de la population de lymphocytes T expansés, à condition que le facteur d'expansion soit supérieur à 10 fois.

**FIG. 1**

FIG. 2

FIG. 3

FIG. 4

FIG. 5

FIG. 6

FIG. 7

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8383099 B
- US 9074185 B
- US 6410319 B
- US 4690915 A
- US 20160187351 A
- US 20170165335 A
- US 20170035807 A
- US 20160280759 A
- US 20160287687 A
- US 20160346371 A
- US 20160368965 A
- US 20170022251 A
- US 20170002055 A
- US 20170029486 A
- US 20170037089 A
- US 20170136108 A
- US 20170101473 A

- US 20170096461 A
- US 20170165337 A
- US 20170189505 A
- US 20170173132 A
- US 20170296640 A
- US 20170253633 A
- US 20170260249 A
- US 20180051080 A
- US 20180164315 A
- US 20170267738 A
- US 20170312350 A
- US 20180161396 A
- US 20180162922 A
- US 20180273602 A
- US 20190002556 A
- US 20180135039 A

**Non-patent literature cited in the description**

- Remington's Pharmaceutical Sciences. 1980